# EUROPEAN PATENT SPECIFICATION

(11) **EP 3 941 488 B1**
(45) Date of publication and mention of the grant of the patent: **14.05.2025**
(21) Application number: 20720187.2
(22) Date of filing: 20.03.2020
(51) Int. Cl.: A61K 39/00, A61K 48/00, C12N 5/078, C12N 5/00, C07K 16/28, C12N 5/0783, C07K 14/725, C12N 15/90, A61P 35/00

(54) **METHODS FOR ENHANCING TCR ALPHA BETA+ CELL DEPLETION EFFICIENCY**
VERFAHREN ZUR STEIGERUNG DER ABREICHERUNGSEFFIZIENZ VON TCR-ALPHA-BETA+-ZELLEN
MÉTHODES D'AMÉLIORATION DE L'EFFICACITÉ DE DÉPLÉTION DE TCR ALPHA BETA+

(30) Priority: 21.03.2019 US 201962821768 P
(43) Date of publication of application: 26.01.2022
(73) Proprietor: Allogene Therapeutics, Inc., South San Francisco, California 94080 (US)
(72) Inventor: NI, Yajin, San Francisco, CA 94080 (US); NING, Hongxiu, San Francisco, CA 94080 (US); LEE, Janet M., San Francisco, CA 94080 (US); LEONARD, Mark W., San Francisco, CA 94080 (US)
(74) Representative: Cooley (UK) LLP
(86) International application number: PCT/US2020/024059
(87) International publication number: WO 2020/191378

(56) References cited:
- WO-A1-2013/074916
- LAURENT POIROT ET AL: "Multiplex Genome-Edited T-cell Manufacturing Platform for ?Off-the-Shelf? Adoptive T-cell Immunotherapies", CANCER RESEARCH, vol. 75, no. 18, 16 July 2015 (2015-07-16), US, pages 3853 - 3864, XP055568648, ISSN: 0008-5472, DOI: 10.1158/0008-5472.CAN-14-3321
- P R GORDON ET AL: "A large-scale method for T cell depletion: towards graft engineering of mobilized peripheral blood stem cells", BONE MARROW TRANSPLANTATION, vol. 30, no. 2, 1 January 2002 (2002-01-01), pages 69 - 74, XP055362192, DOI: 10.1038/sj.bmt.1703619
- ANONYMOUS: "Anti-Human,TCR-[alpha][beta],FITC/TCR-[gamma][delta],PE/CD3,PerCP-Cy(TM)5.5,RUO,(GMP) - 335802 | BD Biosciences-US", 16 February 2019 (2019-02-16), XP055700271, Retrieved from the Internet <URL:http://web.archive.org/web/20190216044323/https://www.bdbiosciences.com/us/reagents/research/clinical-research---ruo-gmp/multicolor-cocktails/3---color-cocktails/anti-human-tcr-alphabeta-fitctcr-gammadelta-pecd3-percp-cytrade55-wt31-11f2-sk7-also-known-as-leu-4/p/335802> [retrieved on 20200602]
- MICHAEL E. BIRNBAUM ET AL: "Molecular architecture of the [alpha][beta] T cell receptor-CD3 complex", PROCEEDINGS OF THE NATIONAL ACADEMY OF SCIENCES, vol. 111, no. 49, 24 November 2014 (2014-11-24), pages 17576 - 17581, XP055619343, ISSN: 0027-8424, DOI: 10.1073/pnas.1420936111
- JUANJUAN ZHAO ET AL: "Universal CARs, universal T cells, and universal CAR T cells", JOURNAL OF HEMATOLOGY & ONCOLOGY, vol. 11, no. 1, 27 November 2018 (2018-11-27), XP055682825, DOI: 10.1186/s13045-018-0677-2
- JANE RASAIYAAH ET AL: "TCR[alpha][beta]/CD3 disruption enables CD3-specific antileukemic T cell immunotherapy", JCI INSIGHT, vol. 3, no. 13, 12 July 2018 (2018-07-12), XP055610826, DOI: 10.1172/jci.insight.99442

## Description

### TECHNICAL FIELD

The instant disclosure relates to methods for depleting cells expressing an endogenous TCR (e.g., TCRαβ) from a population of engineered immune cells, including those comprising chimeric antigen receptors (CARs).

### BACKGROUND

Adoptive transfer of immune cells genetically modified to recognize malignancy-associated antigens is showing promise as a new approach to treating cancer (see, e.g., Brenner et al., Current Opinion in Immunology, 22(2): 251-257 (2010); Rosenberg et al., Nature Reviews Cancer, 8(4): 299-308 (2008)). Immune cells can be genetically modified to express chimeric antigen receptors (CARs) (see, e.g., Eshhar et al., Proc. Natl. Acad. Sci. USA, 90(2): 720-724 (1993), and Sadelain et al., Curr. Opin. Immunol, 21(2): 215-223 (2009)). Immune cells that comprise CARs, e.g. CAR-T cells (CAR-Ts), are engineered to endow them with antigen specificity while retaining or enhancing their ability to recognize and kill a target cell, such as a cancer cell. However, the potential for developing graft versus host disease (GvHD) or host versus graft disease (HvGD) represents a major safety or effectiveness obstacle for the widespread use of engineered allogeneic CAR-T cells in cancer therapy. Thus, there is a need for developing effective methods of reducing the risk of GvHD and HvGD, notably for allogeneic therapies.

### SUMMARY

Described herein are improved methods for TCR+ (TCRαβ+) cell depletion from a population of immune cells, methods for producing a population of immune cells depleted in TCR+ cells, kits comprising reagents therefor, populations of highly pure TCR-(TCRαβ-) cells and methods of treatment employing populations of cells prepared using the disclosed methods. For example, described herein are methods that are particularly suitable for TCR+ cell depletion, which can be used for manufacture of cells useful in allogeneic cell therapies by reducing the risk of GvHD, and can be used in therapies employing chimeric antigen receptors (e.g., allogeneic CAR-T cell therapy).

In an aspect, the invention is a method of producing a population of immune cells depleted of immune cells expressing an endogenous TCR as defined in claim 1. The method can further comprise labeling the population of immune cells with an anti-CD52 antibody; and depleting anti-CD52 antibody labeled immune cells from the population of immune cells. Additionally, the method can further comprise labeling the population of immune cells with an anti-CD52 antibody; and separating anti-CD52 antibody labeled immune cells from unlabeled immune cells.

In another aspect, the invention is a method of depleting cells expressing an endogenous TCR from a population of immune cells as defined in claim 2.

In embodiments, the anti-TCR antibody, anti-CD3 antibody, and/or the anti-CD52 antibody is biotin conjugated and the method can further comprise contacting the labeled cells with an agent that specifically recognizes biotin. The agent that specifically recognizes biotin can be selected from the group consisting of an anti-biotin antibody, avidin and streptavidin and can be conjugated to a magnetic bead, an agarose bead, an acoustic wave particle, a plastic welled plate, a glass welled plate, a ceramic welled plate, a column, a cell culture bag, or a membrane. In other embodiments the anti-TCR antibody, anti-CD3 antibody, and/or the anti-CD52 antibody is directly conjugated to a magnetic bead, an agarose bead, an acoustic wave particle, a plastic welled plate, a glass welled plate, a ceramic welled plate, a column, a cell culture bag, or a membrane.

In methods which employ separation the separating can be achieved using one of a magnetic separation, or acoustic wave separation.

In embodiments the immune cells of the disclosed methods are allogeneic immune cells, and the immune cells can be genetically modified to reduce or eliminate expression of endogenous TCR, endogenous CD52 or both endogenous TCR and endogenous CD52, the reduction or elimination of endogenous TCR or endogenous CD52 or both endogenous TCR and endogenous CD52 expression is achieved using a TALEN, CRISPR/Cas9, a zinc finger nuclease (ZFN), a MegaTAL, a meganuclease, Cpf1, homologous recombination, or a single stranded oligodeoxynucleotide (ssODN).

The immune cells of the disclosed methods can be engineered immune cells expressing a chimeric antigen receptor.

In some embodiments, the population of cells that is depleted of cells expressing an endogenous TCR comprises no more than 1.0% TCR+ cells, no more than 0.9% TCR+ cells, no more than 0.8% TCR+ cells, no more than 0.7% TCR+ cells, no more than 0.6% TCR+ cells, no more than 0.5% TCR+ cells, no more than 0.4% TCR+ cells, no more than 0.3% TCR+ cells, no more than 0.2% TCR+ cells, or no more than 0.1% TCR+ cells. In some embodiments the population of cells that is depleted of cells expressing an endogenous TCR comprises between 0.01-0.001% TCR+ cells immediately after depletion. In some embodiments the population of cells that is depleted of cells expressing an endogenous TCR comprises between 0.1-0.01% TCR+ cells after 1-10 days of culturing post depletion; in some embodiments the population of cells that is depleted of cells expressing an endogenous TCR comprises less than 0.1-1.0% TCR+ cells after 1 day of culturing post depletion. In some embodiments the population of cells that is depleted of cells expressing an endogenous TCR comprises less than 0.1-1.0% TCR+ cells after 10 days of culturing post depletion.

In embodiments of the disclosed methods the immune cell is a T cell.

Disclosed herein for reference is a kit for depleting cells expressing an endogenous TCR from a population of immune cells comprising an anti-TCR antibody and an anti-CD3 antibody. The kit can further comprise an anti-CD52 antibody. In the disclosed kits, the anti-TCR antibody, anti-CD3 antibody, and/or the anti-CD52 antibody is conjugated with an agent that specifically recognizes biotin; the agent that specifically recognizes biotin can be selected from the group consisting of an anti-biotin antibody, streptavidin, and avidin. The agent can be conjugated to a magnetic bead, an agarose bead, an acoustic wave particle, a plastic welled plate, a glass welled plate, a ceramic welled plate, a column, a cell culture bag, or a membrane.

In the disclosed kits the anti-TCR antibody, anti-CD3 antibody, and/or the anti-CD52 antibody is conjugated directly to a support, and the support can be one of a magnetic bead an agarose bead, an acoustic wave particle, a plastic welled plate, a glass welled plate, a ceramic welled plate, a column, a cell culture bag, or a membrane.

Further provided for reference is a method of treating a solid or hematologic cancer in a subject in need thereof comprising administering to the subject a therapeutic amount of a population of immune cells depleted of immune cells expressing an endogenous TCR prepared using the disclosed methods or a population of engineered T cells prepared using the disclosed methods.

The methods provided herein represent a significant advance in allogenic therapy. Thus, also provided for reference is a method of treating a solid or hematologic cancer in a subject in need thereof comprising administering to the subject the population of immune cells depleted of immune cells expressing an endogenous TCR, prepared using any of the disclosed methods provided herein.

### BRIEF DESCRIPTION OF THE DRAWINGS

FIG.1 depicts a schematic representation of an allogenic CAR T manufacturing processes with different unit operation scenarios.
FIG. 2A depicts residual TCR+ cell frequencies in the depleted cell populationson various days post depletion, arrows indicate the fold changes in TCR+ % of different depletion methods (employing different antibody combinations) compared to the control method (employing only anti-TCR antibody) on day 2 post-depletion compared to Day 0 post-depletion
FIG. 2B depicts residual TCR+ cell frequencies in the depleted cell populations, arrows indicate the fold changes in TCR+ % on day 9 post-depletion compared to Day 0 post-depletion between different depletion methods when compared to the control method.
FIG. 3A depicts flow cytometry plots of TCR+ cell depletion efficiencies using different antibodies on Day 0 and Day 1 post depletion measured by anti-TCR antibody staining.
FIG. 3B depicts residual TCR+ cell frequencies in the depleted cell populations on day 0 and day 1 post depletion in numerical bar manner.
FIG. 4 depicts flow cytometry plots demonstrating residual TCR+ and CD3+ frequencies in the depleted populations detected by anti-TCR and/or anti-CD3 antibodies single or double staining on day 0 and day 1 post depletion.
FIG. 5A depicts flow cytometry plots of TCR+, CD3+, CD3+/TCR- and CD3/TCR+ frequencies in the depleted cell populations measured by anti-TCR and/or anti-CD3 antibody single or double staining on Day 1 post depletion before and after freezing.
FIG. 5B depicts TCR+ cell frequencies in the depleted cell populations measured by anti-TCR antibody staining on Day 1 post depletion before and after freezing in numerical bar manner.
FIG. 5C depicts CD3+ cell frequencies in the depleted cell populations measured by anti-CD3 antibody staining on Day 1 post depletion before and after freezing in numerical bar manner.
FIG. 5D depicts CD3+/TCR- cell frequencies in the depleted cell populations measured by anti-TCR and anti-CD3 antibody double staining on Day 1 post depletion before and after freezing in numerical bar manner.
FIG. 5E depicts CD3+/TCR+ cell frequencies in the depleted cell populations measured by anti-TCR and anti-CD3 antibody double-staining on Day 1 post depletion before and after freezing in numerical bar manner.
FIG. 6 depicts flow cytometry plots for TCR+ frequency using anti-TCR antibody staining during 10-day post depletion culturing.
FIG. 7 depicts flow cytometry plots for CD3+ frequency using anti-CD3 antibody staining during 10-day post depletion culturing.
FIG. 8 depicts flow cytometry plots for double TCR+ and CD3+ frequency using both anti-TCR and anti-CD3 antibody staining during 10-day post depletion culturing. FIG. 9A depicts TCR+ cell frequency using anti-TCR antibody staining during post depletion culturing in numerical bar manner.
FIG. 9B depicts CD3+ cell frequency using anti-CD3 antibody staining during post depletion culturing in numerical bar manner.
FIG. 9C depicts CD3+/TCR- cell frequency using both anti-TCR and anti-CD3 antibody staining during post depletion culturing in numerical bar manner.
FIG. 9D depicts CD3+/TCR+ cell frequency using both anti-TCR and anti-CD3 antibody staining during post depletion culturing in numerical bar manner.
FIG. 10A depicts cell growth status in term of viable cell density during 10-day post depletion culturing.
FIG. 10B depicts cell growth status in term of cell viability during 10-day post depletion culturing.
FIG. 10C depicts cell growth status in term of cell diameter during 10-day post depletion culturing.
FIG. 10D depicts cell growth status in term of total cell expansion fold during 10-day post depletion culturing.
FIG. 11A depicts flow cytometry plots demonstrating CD52 depletion efficiencies by usingdifferent antibodies on day 0 and day 1 post depletion measured by anti-CD52 staining.
FIG. 11B depicts residual CD52+ cell frequencies in the depleted cell populations on day 0 and day 1 post depletion measured by anti-CD52 antibody staining in numerical bar manner.
FIG. 12 depicts flow cytometry plots for residual CD52+ cell frequencies during 10-day post depletion culturing monitored by anti-CD52 antibody staining.

### DETAILED DESCRIPTION

Provided herein, are improved methods for robust TCRα/β+ cell depletion, which can minimize the GVHD risk in patients receiving allogeneic CAR T cell therapy, and populations of cells prepared using the disclosed methods. In one aspect, the present disclosure provides methods of increasing TCR+ cell depletion efficiency to significantly reduce residual levels of TCR+ cells in TCR- cell populations.

As used herein, the terms "a" and "an" are used to mean one or more. For example, a reference to "a cell" or "an antibody" means "one or more cells" or "one or more antibodies."

As used herein, the term "TCR-depleted" when used in reference to a population of cells generated using the methods provided in the instant disclosure, means a population of cells that comprises fewer cells expressing endogenous a TCRα/β heterodimer than a population of cells that is collected from a donor. For example, a population of TCR depleted cells (TCRα/βdepleted cells) can comprise 10%, 9%, 8%, 7%, 6%, 5%, 4%, 3%, 2%, 1% or less than 1% of cells expressing an endogenous TCRα/β complex.

As used herein, the term "TCR+" and "TCR" when used in reference to a cell or population of cells, including populations generated using the methods provided in the instant disclosure, refers to cells that express at least an endogenous TCRα/β heterodimer, although one or more components of the CD3 complex may or may not be expressed on the cell surface.

As used herein, the term "TCR-" when used in reference to a cell or population of cells, including populations generated using the methods provided in the instant disclosure, refers to cells that lack at least a TCRα/β heterodimer, although one or more components of the CD3 complex may or may not be expressed on the cell surface.

As used herein, the term "anti-TCR antibody" and "anti-TCR," which are used interchangeably, refer to an antibody that binds and recognizes the human TCRα chain alone, the human TCRβ chain alone or the human TCRα/β heterodimer. An anti-TCR antibody can be a murine, human or humanized antibody.

As used herein, the term "anti-CD3 antibody" and "anti-CD3," which are used interchangeably refer to an antibody that binds and recognizes and binds the human CD3γ chain, the human CD3δ chain, the CD3ζ chain, the CD3ε chain or a human CD3 complex comprising two or more components of the normal human ε/ε/ζ/ζ/γ/δ CD3 complex. An anti-CD3 antibody can be a murine, human or humanized antibody.

The terms "patient" and "subject" are used interchangeably and include human and non-human animal subjects as well as those with formally diagnosed disorders, those without formally recognized disorders, those receiving medical attention, those at risk of developing the disorders, etc.

The term "treat" and "treatment" includes therapeutic treatments, prophylactic treatments, and applications in which one reduces the risk that a subject will develop a disorder or other risk factor. Treatment does not require the complete curing of a disorder and encompasses examples in which one reduces symptoms or underlying risk factors. The term "prevent" does not require the 100% elimination of the possibility of an event. Rather, it denotes that the likelihood of the occurrence of the event has been reduced in the presence of the compound or method.

Engineered TCR- cell-based allogeneic CAR-T products exemplify a strategy for generating next generation CAR-T therapeutics. However, the potential immune responses such as Graft Versus Host Disease (GvHD) risk and Host versus Graft Disease (HvGD) represents one of the significant safety or effectiveness concerns for allogenic CAR-T cell therapy. GvHD results from donor derived T-cells recognizing HLA mismatch via the T cell αβ receptor (TCRαβ) and having the potential to attack the patient's tissues. GVHD and HvGD can be serious and even fatal, even in the HLA matched donor setting, as minor mismatches can still provoke an immune response.

Current methods for removing TCR+ cells (TCRαβ+ cell depletion) such as the CliniMACS Prodigy^{®} and TCR reagent kit, employs an anti-TCR antibody alone to separate TCR+ cells (TCRαβ+ cells) from TCR- cells (TCRαβ- cells) using an anti-TCR antibody and may achieve a 99% or greater TCR- cell purity in the final drug product (see, e.g., Radestad et al, (2014) J Immunol Res, Vol 2014: 578741). However, less than 1% residual levels of TCR+ cells present in the TCR- final drug product can still present the risk of GvHD associated with allogeneic CAR-T cell therapy, especially at high dose levels.

The instant disclosure provides methods for producing a population of engineered immune cells depleted in immune cells expressing an endogenous TCR, the disclosed method comprises labeling a population of immune cells with an anti-TCR antibody and an anti-CD3 antibody; and separating anti-TCR antibody labeled immune cells and anti-CD3 antibody labeled immune cells from the population of engineered immune cells.

The instant disclosure also provides a method of depleting cells expressing an endogenous TCR from a population of immune cells, the disclosed method comprising labeling the population of immune cells with an anti-TCR antibody and an anti-CD3 antibody; separating anti-TCR antibody labeled immune cells and anti-CD3 antibody labeled immune cells from unlabeled immune cells; and collecting the unlabeled immune cells, thereby obtaining a population of immune cells that are depleted of cells expressing an endogenous TCR. The method can also be adapted to comprise a population of immune cells labeled with anti-TCR antibodies and one or more antibodies directed to any other target(s) of interest other than CD3 (e.g., MHC1, MHC2, CD52 or PD1).

In one aspect, the present disclosure provides methods of depleting TCRαβ+ cells comprising contacting a population of immune cells with a TCR antibody and a CD3 antibody. The method can also be adapted to comprise a population of immune cells labeled with anti-TCR antibodies and one or more antibodies directed to any other target(s) of interest other than CD3 (e.g., MHC1, MHC2, CD52 or PD1.

In yet another aspect, the present disclosure provides a method of depleting TCRαβ+ cells comprising contacting a population of immune cells with an anti-TCR antibody, an anti-CD3 antibody, and an anti-CD52 antibody.

The methods described herein apply anti-TCR and anti-CD3 antibodies together for TCRαβ+ cell depletion, which is of particular use for generating an engineered TCRαβ- cell-based allogeneic CAR T product. Prior to the present disclosure, clinical scale depletion of TCR+ cells in engineered T cells (not an un-modified T cell product) to a level of 99.9%-99.99% TCR- cell purity, leaving a 0.1%-0.01% TCR+ cell residual was extremely difficult and represented a challenge for the development of allogenic therapies. The instant disclosure provides a solution to this problem.

Achieving this kind of low level residual TCR+ cells (e.g., in the range of 0.1%-0.01% TCR+ cells measured 1 day after depletion) represents a significant advancement of engineered allogeneic CAR T products (e.g., TCRα/β- CAR T cells) and provides an enhanced clinical benefit to patients by decreasing the potential for GvHD.

Effective methods of reducing the risk of GvHD (and HvGD), as well as enhancing anti-tumor potency can be particularly useful for the manufacture of effective, safe and pure engineered allogeneic immune cells (e.g., allogeneic CAR-T cells expressing a CAR targeted to a cancer antigen). In some embodiments, the present disclosure provides methods for producing allogeneic CAR-T cells and products that reduce the risk of, or prevent the development of, GvHD and/or HvGD. In some embodiments the engineered allogeneic CAR-T cells have enhanced anti-tumor activities, against solid tumors or hematologic cancers. In some embodiments, the CAR-T cells comprise a population of cells that are modified to reduce or eliminate expression of one or more of endogenous TCR, CD52, MHC1, MHC2, or PD1 gene expression. Thus, in some embodiments, the CAR-T cells comprise a population that is depleted of cells that are TCR+, CD52+, MHC1+, MHC2+, and/orPD1+.

Provided herein are methods of depleting populations of cells expressing one or more of endogenously expressed TCR, CD3, CD52, MHC1, MHC2, or PD1 (e.g., cells that are TCR+, CD3+, CD52+, MHC1+, MHC2+ and/or PD1+), from a population of engineered immune cells, and kits comprising antibody reagents therefor.

### T cell receptor signaling

The T cell receptor complex (TCR, as described herein) is an antigen receptor molecule on the surface of T cells, responsible for the recognition of antigen presented to T cells by MHC molecules leading potentially to the activation of the T cell and an immune response to the antigen. The human T cell receptor is a heterodimer consisting of two transmembrane heterodimeric glycoprotein chains, the α and β chains (there are also a small proportion of γδ chains) each with two domains, which are linked by a disulfide bond. Due to the short cytoplasmic tail of the TCR, it cannot directly signal when it binds to a peptide-MHC complex. Instead the TCR is associated with a group of signaling molecules collectively called CD3 which transmit an intracellular signal when the TCR binds to a peptide-MHC complex.

CD3 is made up of one γ and δ and two ε molecules which all have in their extracellular domains some limited sequence homology to the immunoglobulin domain. These molecules have small cytoplasmic domains and transmembrane domains with negatively charged residues. In the membrane, these negatively charged residues form salt bridges with the positively charged residues in the transmembrane region of the TCR. The TCR-CD3 receptor complex is completed by two other invariant proteins ζ and η which form dimers linked by disulfide bonds. At the T cell surface therefore, the TCR-CD3 complex is expressed as a αβ (or γδ) heterodimer, in association with CD3γε and CD3δε dimers with an intracellular ζζ homodimers or a ζη heterodimer.

Without wishing to be bound by theory, some researchers believe that if TCR expression is disrupted, CD3 surface expression may also be abolished. However, as described herein, it was unexpectedly discovered that residual CD3 surface expression may still be indicative of residual TCR+ cells and TCR+ depletion efficiency can be improved by using an anti-CD3 antibody in combination with an anti-TCR antibody.

### Methods of Sorting and Depleting Immune Cells

As described herein, in one aspect the present disclosure provides a method of depleting cells expressing an endogenous TCRα/β dimer from a population of immune cells, the method comprising labeling the population of cells with an anti-TCR antibody and an anti-CD3 antibody (which labeling can be performed sequentially or at the same time in a single operation), separating the anti-TCR and anti-CD3 labeled cells from the unlabeled cells, and collecting the unlabeled cells, thereby obtaining a population of cells that are depleted of cells expressing an endogenous TCR.

Initially, engineered immune cells modified so as to be deficient for the endogenous TCRα or TCRβ gene are exposed to a TCR depletion reagent. The TCR depletion reagent comprises an antibody targeting the TCRα polypeptide, TCRβ polypeptide, or TCRα/β heterodimer endogenously expressed on the surface of immune cells. As described herein, TCR depletion using an anti-TCR antibody in combination with an anti-CD3 antibody (and optionally an anti-CD52 antibody) antibody unexpectedly increases the TCR+ depletion efficiency compared to depletions performed with an anti-TCR antibody alone.

The anti-TCR antibody, anti-CD3 antibody, or the anti-CD52 antibody (or any other antibody directed to a target of interest to a TCR depletion operation) can be conjugated to biotin to facilitate further labeling and/or separation using a secondary antibody (e.g., an anti-biotin antibody) conjugated either directly or indirectly with a magnetic depletion reagent such as magnetic depletion agent such as magnetic microbeads (nanoparticles that are generally, but not necessarily, about 50nm in diameter) or any other surface, such as an an agarose bead, an acoustic wave particle, a plastic welled plate, a glass welled plate, a ceramic welled plate, a column, a cell culture bag, or a membrane. When magnetic microbeads are used, the microbeads facilitate separation of the TCR+ cells from the TCR- cells; when contacted with a magnetic column the TCR+ cells can be retained on the column while unlabeled TCR- cells pass through to a collection bag. Acoustic wave particles can facilitate separation of TCR+ from the TCR- cells when exposed to an acoustic wave. While an anti-biotin antibody is provided in the context of the disclosed method, other biotin-binding partners such as streptavidin, avidin, and other proteins that recognize biotin can be employed in lieu of an anti-biotin antibody in all of the methods provided herein.

In some embodiments, provided cells may be optionally sorted for other cell surface markers. For example, a subset of a population of immune cells can comprise engineered immune cells expressing an antigen-specific CAR which itself comprises one ore more epitopes specific for one or more monoclonal antibodies (e.g., exemplary mimotope sequences; see, e.g., WO2016/120216). The method comprises contacting the population of immune cells with a monoclonal antibody specific for the epitopes and selecting the immune cells that bind to the monoclonal antibody to obtain a population of cells enriched in engineered immune cells expressing an antigen-specific CAR.

In some embodiments, said monoclonal antibody specific for said epitope is optionally conjugated to a fluorophore. In this embodiment, the step of selecting the cells that bind to the monoclonal antibody can be done by Fluorescence Activated Cell Sorting (FACS).

In some embodiments, said monoclonal antibody specific for said epitope is optionally conjugated to a magnetic particle. In this embodiment, the step of selecting the cells that bind to the monoclonal antibody can be done by Magnetic Activated Cell Sorting (MACS).

In some embodiments, the monoclonal antibody used in the method for sorting immune cells expressing the CAR is chosen from alemtuzumab, ibritumomab tiuxetan, muromonab-CD3, tositumomab, abciximab, basiliximab, brentuximab vedotin, cetuximab, infliximab, rituximab, bevacizumab, certolizumab pegol, daclizumab, eculizumab, efalizumab, gemtuzumab, natalizumab, omalizumab, palivizumab, ranibizumab, tocilizumab, trastuzumab, vedolizumab, adalimumab, belimumab, canakinumab, denosumab, golimumab, ipilimumab, ofatumumab, panitumumab, QBEND-10 and/or ustekinumab. In some embodiments, said mAb is rituximab. In another embodiment, said mAb is QBEND-10.

Flow cytometry can be used to quantify specific cell types within a population of cells. In general, flow cytometry is a method for quantitating components or structural features of cells primarily by optical means. Since different cell types can be distinguished by quantitating structural features, flow cytometry and cell sorting can be used to count and sort cells of different phenotypes in a mixture.

A flow cytometry analysis involves two primary steps: 1) labeling selected cell types with one or more detectable markers, and 2) determining the number of labeled cells relative to the total number of cells in the population. In some embodiments, the method of labeling cell types includes binding labeled antibodies to markers expressed by the specific cell type. The antibodies may be either directly labeled with a fluorescent compound or indirectly labeled using, for example, a fluorescent-labeled second antibody which recognizes the first antibody.

In some embodiments of the disclosed methods, sorting or separating TCR+ cells, optionally expressing a CAR, from TCR- cells can be achieved using Magnetic-Activated Cell Sorting (MACS). Magnetic-activated cell sorting (MACS) is a method for separation of various cell populations depending on their surface antigens (CD molecules) by using superparamagnetic nanoparticles and columns. MACS can be used to obtain a very pure cell population. Cells in a single-cell suspension can be magnetically labeled with microbeads. The sample is applied to a column composed of a ferromagnetic material, which is covered with a coating not disruptive to cells, thus allowing fast and gentle separation of cells. The unlabeled cells pass through the column while the magnetically-labeled cells are retained within the column. The flow-through can be collected as the unlabeled cell fraction. After a washing step, the column is removed from the separator, and the magnetically labeled cells are eluted from the column.

A detailed protocol for the purification of specific cell population such as T-cell can be found in Basu S et al. (2010). (Basu S, Campbell HM, Dittel BN, Ray A. Purification of specific cell population by fluorescence activated cell sorting (FACS), J. Vis. Exp. (41): 1546).

In some embodiments of the disclosed methods, sorting or separating TCR+ cells, optionally expressing a CAR, from TCR- cells can be achieved using acoustic wave separation in lieu of magnetic-based separation methods. While not wishing to be bound by theory, it is understood that acoustic wave separation relies on a three-dimensional standing wave to separate components of a mixture. In the context of the disclosed methods, an antibody such as an anti-TCR antibody, an anti-CD52 antibody or an anti-CD3 antibody can be conjugated to a surface, such as an acoustic wave particle. An acoustic wave particle can be a bead. In an embodiment cells are exposed to acoustic wave particles bearing one or more of an anti-TCR antibody, an anti-CD52 antibody or an anti-CD3 antibody, associating the acoustic wave particle with any cells expressing the target of interest. The cells are then placed in an acoustic chamber and exposed to an acoustic wave. Given the different properties of the bead-associated cells and cells that were not labeled with the antibody-bead particles, the acoustic wave separates the labled and unlabled cells, which can be collected while labled cells (e.g., TCR+ or CD52+ cells) can be divert away from the labeled cells.

### Immune Cells

Cells suitable for the TCR+ cell depletion methods described herein are immune cells.

Prior to the *in vitro* manipulation or genetic modification (e.g., as described herein), immune cells for use in methods described herein can be obtained from a subject. Cells can be obtained from a number of non-limiting subject-based sources, including peripheral blood mononuclear cells (PBMCs), bone marrow, lymph node tissue, cord blood, thymus tissue, tissue from a site of infection, ascites, pleural effusion, spleen tissue, and tumors. In some embodiments, any number of T cell lines available and known to those skilled in the art, may be used. In some embodiments, cells can be derived from a healthy donor or from a patient diagnosed with cancer. In some embodiments, cells can be part of a mixed population of cells which present different phenotypic characteristics.

In embodiments, immune cells are obtained from a subject who will ultimately receive the engineered immune cells (i.e., autologous therapy). In embodiments, immune cells are obtained from a donor, who is a different individual from the subject who will receive the engineered immune cells (i.e., allogeneic therapy).

Cells can be obtained from the circulating blood of an individual by apheresis. The apheresis product typically contains lymphocytes, including T cells, monocytes, granulocytes, B cells, other nucleated white blood cells, red blood cells, and platelets. In certain embodiments, the cells collected by apheresis can be washed to remove the plasma fraction and placed in an appropriate buffer or media for subsequent processing.

PBMCs can be used directly for genetic modification to generate engineered immune cells (such as CARs or TCRs) using methods as described herein. In certain embodiments, after isolating the PBMCs, T lymphocytes can optionally be further isolated to generate a population comprising only T cells, and both cytotoxic and helper T lymphocytes can be further sorted into naive, memory, and effector T cell subpopulations either before or after genetic modification and/or expansion.

In certain embodiments, T cells can be isolated from PBMCs by lysing the red blood cells and depleting the monocytes, for example, using centrifugation through a PERCOLL^{®} gradient. Specific subpopulations of T cells, such as CD28+, CD3+, CD4+, CD8+, CD25+, CD62L+, CD5+, CD45RA-, CCR7+, CD95+, IL2Rβ+and CD45RO+ T cells can be further isolated by positive or negative selection techniques known in the art. For example, enrichment of a T cell population by negative selection can be accomplished with a combination of antibodies directed to surface markers unique to the negatively selected cells. One method for use herein is cell sorting and/or selection via negative magnetic immunoadherence or flow cytometry that uses a cocktail of monoclonal antibodies directed to cell surface markers present on the cells negatively selected. For example, to enrich for CD4+cells by negative selection, a monoclonal antibody cocktail typically includes antibodies to CD14, CD20, CD11b, CD16, HLA-DR, and CD8. Flow cytometry and cell sorting may also be used to isolate cell populations of interest for use in the present disclosure.

In some embodiments, a population of T cells is enriched for CD8+ cells either before or after applying the depletion methods provided herein.

In some embodiments, a population of T cells is enriched for CD4+ cells either before or after applying the delpletion methods provided herein.

In some embodiments, populations or CD4+ and/or CD8+ cells can be further sorted into naive, stem cell memory, central memory, effector memory and effector cells by identifying cell surface antigens that are associated with each of these types of cells. In some embodiments the expression of phenotypic markers for naive T cells include CD45RA+, CD95-, IL2Rβ-, CCR7+, and CD62L+. In some embodiments the expression of phenotypic markers for stem cell memory T cells include CD45RA+, CD95+, IL2Rβ+, CCR7+, and CD62L+. In some embodiments the expression of phenotypic markers for central memory T cells include CD45RO+, CD95+, IL2Rβ+, CCR7+, and CD62L+. In some embodiments the expression of phenotypic markers for effector memory T cells include CD45RO+, CD95+, IL2Rβ+, CCR7-, and CD62L-. In some embodiments the expression of phenotypic markers for T effector cells include CD45RA+, CD95+, IL2Rβ+, CCR7-, and CD62L-. Thus, CD4+ and/or CD8+ T helper cells can be sorted into naive, stem cell memory, central memory, effector memory and T effector cells by identifying cell populations that have cell surface antigens. In specific embodiments the disclosed methods can be employed to enhance depletion of one or more of these subsets of T cells.

It will be appreciated that PBMCs can further include other cytotoxic lymphocytes such as NK cells or NK T cells. An expression vector carrying the coding sequence of a chimeric receptor as disclosed herein can be introduced into a population of human donor T cells, NK cells or NK T cells. Standard procedures can be used for cryopreservation of T cells expressing the CAR for storage and/or preparation for use in a human subject. In one embodiment, the *in vitro* transduction, culture and/or expansion of T cells are performed in the absence of non-human animal derived products such as fetal calf serum and fetal bovine serum. In vaious embodiments a crypreservative media can comprise, for example, CryoStor^{®} CS2, CS5, or CS10 or other medium comprising DMSO, or a medium that does not comprise DMSO.

### Engineered Immune Cells

The TCR+ cell depletion methods described herein can be particularly useful in manufacturing immune cell therapies for treating cancer, including therapies comprising engineered immune cells (e.g., CAR-T cells).

Engineered immune cells can be allogeneic or autologous, and the disclosed methods can be applied in either an autologous or allogeneic therapy.

In some embodiments, an engineered immune cell (or population thereof) is or comprises a T cell (e.g., inflammatory T-lymphocyte, cytotoxic T-lymphocyte, regulatory T-lymphocyte, helper T-lymphocyte, tumor infiltrating lymphocyte (TIL)), NK cell, NK-T-cell, TCR-expressing cell, dendritic cell, killer dendritic cell, a mast cell, or a macrophage. In some embodiments, the engineered immune cell can be derived from the group consisting of CD4+ T-lymphocytes, CD8+ T-lymphocytes or a combination thereof. In some exemplary embodiments, the engineered immune cell is a T cell. T cells can also be γ/δ T cells.

In some embodiments, an engineered immune cell can be derived from, for example without limitation, a stem cell. The stem cells can be adult stem cells, non-human embryonic stem cells, more particularly non-human stem cells, cord blood stem cells, progenitor cells, bone marrow stem cells, induced pluripotent stem cells (iPSCs), totipotent stem cells or hematopoietic stem cells. Stem cells can be CD34+ or CD34-.

In some embodiments, the cell is obtained or prepared from peripheral blood. In some embodiments, the cell is obtained or prepared from peripheral blood mononuclear cells (PBMCs). In some embodiments, the cell is obtained or prepared from bone marrow. In some embodiments, the cell is a human cell. In some embodiments, the cell is transfected or transduced by a nucleic acid vector using a method selected from the group consisting of viral (lentiviral or gamma retroviral) transduction, electroporation, sonoporation, biolistics (e.g., Gene Gun), lipid transfection, polymer transfection, nanoparticles, or polyplexes. In some embodiments the cell is a T cell that has been re-programmed from a non-T cell. In some embodiments the cell is a T cell that has been re-programmed from a T cell.

### Binding Agents (including antibodies and fragments thereof)

The disclosed methods comprise the use of an antibody or antigen binding agent (e.g., comprising an antigen binding domain or comprising an antibody or fragment thereof). As discussed below, in various embodiments engineered immune cells can also comprise a binding agent.

As used herein, the term "antibody" refers to a polypeptide that includes canonical immunoglobulin sequence elements sufficient to confer specific binding to a particular target antigen. As is known in the art, intact antibodies as produced in nature are approximately 150 kD tetrameric agents comprised of two identical heavy chain polypeptides (about 50 kD each) and two identical light chain polypeptides (about 25 kD each) that associate with each other into what is commonly referred to as a "Y-shaped" structure. Each heavy chain is comprised of at least four domains (each about 110 amino acids long)- an amino-terminal variable (VH) domain (located at the tips of the Y structure), followed by three constant domains: CHI, CH2, and the carboxy-terminal CH3 (located at the base of the Y's stem). A short region, known as the "switch", connects the heavy chain variable and constant regions. The "hinge" connects CH2 and CH3 domains to the rest of the antibody. Two disulfide bonds in this hinge region connect the two heavy chain polypeptides to one another in an intact antibody. Each light chain is comprised of two domains - an amino-terminal variable (VL) domain, followed by a carboxy-terminal constant (CL) domain. Those skilled in the art are well familiar with antibody structure and sequence elements, recognize "variable" and "constant" regions in provided sequences, and understand that there may be some flexibility in definition of a "boundary" between such domains such that different presentations of the same antibody chain sequence may, for example, indicate such a boundary at a location that is shifted one or a few residues relative to a different presentation of the same antibody chain sequence.

Intact antibody tetramers are comprised of two heavy chain-light chain dimers in which the heavy and light chains are linked to one another by a single disulfide bond; two other disulfide bonds connect the heavy chain hinge regions to one another, so that the dimers are connected to one another and the tetramer is formed. Naturally-produced antibodies are also glycosylated, typically on the CH2 domain. Each domain in a natural antibody has a structure characterized by an "immunoglobulin fold" formed from two beta sheets (e.g., 3-, 4-, or 5- stranded sheets) packed against each other in a compressed antiparallel beta barrel. Each variable domain contains three hypervariable loops known as "complement determining regions" (CDR1, CDR2, and CDR3) and four somewhat invariant "framework" regions (FR1, FR2, FR3, and FR4). When natural antibodies fold, the FR regions form the beta sheets that provide the structural framework for the domains, and the CDR loop regions from both the heavy and light chains are brought together in three-dimensional space so that they create a single hypervariable antigen binding site located at the tip of the Y structure. The Fc region of naturally-occurring antibodies binds to elements of the complement system, and also to receptors on effector cells, including for example effector cells that mediate cytotoxicity. As is known in the art, affinity and/or other binding attributes of Fc regions for Fc receptors can be modulated through glycosylation or other modification. In some embodiments, antibodies produced and/or utilized in accordance with the present invention include glycosylated Fc domains, including Fc domains with modified or engineered such glycosylation.

For purposes of the instant disclosure, in certain embodiments, any polypeptide or complex of polypeptides that includes sufficient immunoglobulin domain sequences as found in natural antibodies can be referred to and/or used as an "antibody," whether such polypeptide is naturally produced (e.g., generated by an organism reacting to an antigen), or produced by recombinant engineering, chemical synthesis, or other artificial system or methodology. In some embodiments, an antibody is polyclonal; in some embodiments, an antibody is monoclonal. In some embodiments, an antibody has constant region sequences that are characteristic of mouse, rabbit, primate, or human antibodies. In some embodiments, antibody sequence elements are humanized, primatized, chimeric, etc, as is known in the art.

Moreover, the term "antibody" as used herein, can refer to any of the art-known or developed constructs or formats for utilizing antibody structural and functional features in alternative presentation. For example, in some embodiments, an antibody utilized in the methods of the instant disclosure is in a format selected from, but not limited to, intact IgA, IgG, IgE or IgM antibodies; bi- or multi- specific antibodies (e.g., Zybodies^{®}, etc.); antibody fragments such as Fab fragments, Fab fragments, F(ab)2 fragments, Fd fragments, and isolated CDRs or sets thereof; single chain variable fragments (scFVs); polypeptide-Fc fusions; single domain antibodies (e.g., shark single domain antibodies such as IgNAR or fragments thereof); camelid antibodies (also referred to herein as nanobodies or VHHs); shark antibodies, masked antibodies (e.g., Probodies^{®}); Small Modular ImmunoPharmaceuticals (SMIPs^{™} ); single chain or Tandem diabodies (TandAb^{®}); VHHs; Anticalins^{®}; Nanobodies^{®} minibodies; BiTE^{®}s; ankyrin repeat proteins or DARPINs^{®}; Avimers^{®}; DARTs; TCR-like antibodies;, Adnectins^{®}; Affilins^{®}; Trans-bodies^{®}; Affibodies^{®}; TrimerX^{®}; MicroProteins; Fynomers^{®}, Centyrins^{®}; and KALBITOR^{®}s. In some embodiments, an antibody may lack a covalent modification (e.g., attachment of a glycan) that it would have if produced naturally. In some embodiments, an antibody may contain a covalent modification (e.g., attachment of a glycan, a payload (e.g., a detectable moiety, a therapeutic moiety, a catalytic moiety, etc.), or other pendant group (e.g., poly-ethylene glycol, etc.).

As used herein, the term "antibody agent" generally refers to an agent that specifically binds to a particular antigen. In some embodiments, the term encompasses any polypeptide or polypeptide complex that includes immunoglobulin structural elements sufficient to confer specific binding. Exemplary antibody agents include, but are not limited to monoclonal antibodies or polyclonal antibodies. In some embodiments, an antibody agent may include one or more constant region sequences that are characteristic of mouse, rabbit, primate, or human antibodies. In some embodiments, an antibody agent may include one or more sequence elements are humanized, primatized, chimeric, etc. as is known in the art. In many embodiments, the term "antibody agent" is used to refer to one or more of the art-known or developed constructs or formats for utilizing antibody structural and functional features in alternative presentation. For example, an antibody agent utilized in accordance with the present invention is in a format selected from, but not limited to, intact IgA, IgG, IgE or IgM antibodies; bi- or multi- specific antibodies (e.g., Zybodies^{®}, etc.); antibody fragments such as Fab fragments, Fab' fragments, F(ab')2 fragments, Fd fragments, and isolated CDRs or sets thereof; single chain Fvs; polypeptide-Fc fusions; single domain antibodies (e.g., shark single domain antibodies such as IgNAR or fragments thereof); cameloid antibodies; masked antibodies (e.g., Probodies^{®}); Small Modular ImmunoPharmaceuticals (SMIPs^{™} ); single chain or Tandem diabodies (TandAb^{®}); VHHs; Anticalins^{®}; Nanobodies^{®} minibodies; BiTE^{®}s; ankyrin repeat proteins or DARPINs^{®}; Avimers^{®}; DARTs; TCR-like antibodies; Adnectins^{®}; Affilins^{®}; Trans-bodies^{®}; Affibodies^{®}; TrimerX^{®}; MicroProteins; Fynomers^{®}, Centyrins^{®}; and KALBITOR^{®}s.

An antibody or antibody agent used in performing the methods of the instant disclosure can be single chained or double chained. In some embodiments, the antibody or antigen binding molecule is single chained. In certain embodiments, the antigen binding molecule is selected from the group consisting of an scFv, a Fab, a Fab', a Fv, a F(ab')₂, a dAb, and any combination thereof.

Antibodies and antibody agents include antibody fragments. An antibody fragment comprises a portion of an intact antibody, such as the antigen binding or variable region of the intact antibody. Antibody fragments include, but are not limited to, Fab, Fab', Fab'-SH, F(ab')₂, Fv, diabody, linear antibodies, multispecific formed from antibody fragments antibodies and scFv fragments, and other fragments. Antibodies also include, but are not limited to, polyclonal monoclonal, chimeric dAb (domain antibody), single chain, Fab, Fa, F(ab')₂ fragments, and scFvs. An antibody can be a whole antibody, or immunoglobulin, or an antibody fragment. Antibody fragments can be made by various techniques, including but not limited to proteolytic digestion of an intact antibody as well as production by recombinant host cells (e.g., E. coli, Chinese Hamster Ovary (CHO) cells, or phage), as known in the art.

In some embodiments, an antibody or antibody agent can be a chimeric antibody (see, e.g., U.S. Pat. No. 4,816,567; and Morrison et al., Proc. Natl. Acad. Sci. USA, 81:6851-6855 (1984)). A chimeric antibody can be an antibody in which a portion of the heavy and/or light chain is derived from a particular source or species, while the remainder of the heavy and/or light chain is derived from a different source or species. In one example, a chimeric antibody can comprise a non-human variable region (e.g., a variable region derived from a mouse, rat, hamster, rabbit, or non-human primate, such as a monkey) and a human constant region. In a further example, a chimeric antibody can be a "class switched" antibody in which the class or subclass has been changed from that of the parent antibody. Chimeric antibodies include antigen-binding fragments thereof.

In some embodiments, a chimeric antibody can be a humanized antibody (See, e.g., Almagro and Fransson, Front. Biosci., 13:1619-1633 (2008); Riechmann et al., Nature, 332:323-329 (1988); Queen et al., Proc. Natl Acad. Sci. USA 86:10029-10033 (1989); U.S. Pat. Nos. 5,821,337, 7,527,791, 6,982,321, and 7,087,409; Kashmiri et al., Methods 36:25-34 (2005); Padlan, Mol. Immunol, 28:489-498 (1991); Dall'Acqua et al., Methods, 36:43-60 (2005); Osbourn et al., Methods, 36:61-68 (2005); and Klimka et al., Br. J. Cancer, 83:252-260 (2000)). A humanized antibody is a chimeric antibody comprising amino acid residues from non-human hypervariable regions and amino acid residues from human FRs. In certain embodiments, a humanized antibody will comprise substantially all of at least one, and typically two, variable domains, in which all or substantially all of the hypervariable regions (e.g., CDRs) correspond to those of a non-human antibody, and all or substantially all of the Framework Regions (FRs) correspond to those of a human antibody. A humanized antibody optionally can comprise at least a portion of an antibody constant region derived from a human antibody.

In some embodiments, an antibody or antibody agent provided herein is a human antibody. Human antibodies can be produced using various techniques known in the art (See, e.g., van Dijk and van de Winkel, Curr. Opin. Pharmacol, 5: 368-74 (2001); and Lonberg, Curr. Opin. Immunol, 20:450-459 (2008)). A human antibody can be one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues. Human antibodies may be prepared using methods well known in the art.

### Chimeric Antigen Receptors (CARs)

As described herein the disclosed methods can be used to deplete TCR+ cells from a population of immune cells. The immune cells can be CAR+ cells and engineered for use in therapeutic applications such as allogeneic therapy. In some embodiments, an engineered immune cell comprises a CAR comprising an extracellular antigen-binding domain. As used herein, chimeric antigen receptors (CARs) comprise proteins that specifically recognize target antigens (e.g., target antigens on cancer cells; cancer antigens). When bound to the target antigen, the CAR can activate the immune cell to attack and destroy the cell bearing that antigen (e.g., the cancer cell). CARs can also incorporate costimulatory domains comprising all or a portion of proteins such as 4-1BB, CD28 or OX 40, and/or a signaling domain such as CD3zeta in order to increase their potency. See Krause et al., J. Exp. Med., Volume 188, No. 4, 1998 (619-626); Finney et al., Journal of Immunology, 1998, 161: 2791-2797, Song et al., Blood 119:696-706 (2012); Kalos et al., Sci. Transl. Med. 3:95 (2011); Porter et al., N. Engl. J. Med. 365:725-33 (2011), and Gross et al., Annu. Rev. Pharmacol. Toxicol. 56:59-83 (2016); U.S. Patent Nos. 7,741,465, and 6,319,494.

Chimeric antigen receptors described herein comprise an extracellular domain, a transmembrane domain, optionally a hinge domain joining the extracellular domain to the transmembrane domain, and an intracellular domain, wherein the extracellular domain comprises an antigen binding domain that specifically binds to the target.

In some embodiments, antigen-specific CARs further comprise a safety switches and/or monoclonal antibody specific-epitope. In some embodiments, an antigen-selective CAR comprises a leader or signal peptide.

### CAR Antigen Binding Domains

As discussed above, CARs described herein comprise an antigen binding domain. An "antigen binding domain" as used herein means any polypeptide that binds a specified target antigen. In some embodiments, an antigen binding domain is a scFv. In some embodiments, the antigen binding domain binds to an antigen on a tumor cell. In some embodiments, the antigen binding domain binds to an antigen on a cell involved in a hyperproliferative disease (e.g., Non-Hodgkin's Lymphoma, Multiple Myeloma, or other solid or hematological cancer). The disclosed methods can be employed to deplete TCR+ cells that are also CAR+ cells, thus providing a population of CAR+/TCR- cells.

In some embodiments, the antigen binding domain of a CAR comprises a variable heavy chain, variable light chain, and/or one or more CDRs described herein. In some embodiments, the antigen binding domain is a single chain variable fragment (scFv), comprising light chain CDRs CDR1, CDR2 and CDR3, and heavy chain CDRs CDR1, CDR2 and CDR3.

An antigen binding domain is said to be "selective" when it binds to one target more tightly than it binds to a second target.

The antigen binding domain of the CAR selectively targets a cancer antigen. In some embodiments, the cancer antigen is selected from EGFRvIII, WT-1, CD20, CD23, CD30, CD38, CD33, CD133, MHC-WT1, TSPAN10, MHC-PRAME, Liv1, ADAM10, CHRNA2, LeY, NKGD2D, CS1, CD44v6, ROR1, Claudin-18.2, Muc17, FAP alpha, Ly6G6D, c6orf23, G6D, MEGT1, NG25, CD19, BCMA, FLT3, CD70, DLL3, CD52 or CD34. In some embodiments, the CAR comprises an antigen binding domain that targets EGFRvIII, WT-1, CD20, CD23, CD30, CD38, CD33, CD133, MHC-WT1, TSPAN10, MHC-PRAME, Liv1, ADAM10, CHRNA2, LeY, NKGD2D, CS1, CD44v6, ROR1, Claudin-18.2, Muc17, FAP alpha, Ly6G6D, c6orf23, G6D, MEGT1, NG25, CD19, BCMA, FLT3, CD70, DLL3, CD52 or CD34.

In some embodiments, the cancer antigen is selected from the group consisting of carbonic anhydrase IX (CAIX), carcinoembryonic antigen (CEA), CDS, CD7, CDIO, CD19, CD20, CD22, CD30, CD33, CD34, CD38, CD41, CD44, CD49f, CD56, CD74, CD123, CD133, CD138, an antigen of a cytomegalovirus (CMV) infected cell (e.g., a cell surface antigen), epithelial glycoprotein (EGP 2), epithelial glycoprotein-40 (EGP-40), epithelial cell adhesion molecule (EpCAM), receptor tyrosine-protein kinases erb- B2,3,4, folate-binding protein (FBP), fetal acetylcholine receptor (AChR), folate receptors, Ganglioside G2 (GD2), Ganglioside G3 (GD3), human Epidermal Growth Factor Receptor 2 (HER-2), human telomerase reverse transcriptase (hTERT), Interleukin-13 receptor subunit alpha-2 (IL-13Ra2), x-light chain, kinase insert domain receptor (KDR), Lewis A (CA19.9), LI cell adhesion molecule (LICAM), melanoma antigen family A, 1 (MAGE-AI), Mucin 16 (Muc-16), Mucin 1 (Muc-1), Mesothelin (MSLN), NKG2D ligands, cancer-testis antigen NY-ESO-1, oncofetal antigen (h5T4), prostate stem cell antigen (PSCA), prostate-specific membrane antigen (PSMA), tumor- associated glycoprotein 72 (TAG-72), vascular endothelial growth factor R2 (VEGF- R2), and Wilms tumor protein (WT-1).

In other embodiments, the disclosure relates to isolated polynucleotides encoding any one of the antigen binding domains described herein. In some embodiments, the disclosure relates to isolated polynucleotides encoding a CAR. Also provided herein are vectors comprising the polynucleotides, and methods of making same.

In some embodiments, a CAR-immune cell (e.g., CAR-T cell) which can form a component of a population of cells generated by practicing the methods of the instant disclosure comprises a polynucleotide encoding a safety switch polypeptide, such as for example RQR8. See, e.g., WO2013153391A. In a CAR-immune cell (e.g., a CAR-T cell) comprising the polynucleotide, the safety switch polypeptide can be expressed at the surface of a CAR-immune cell (e.g., CAR-T cell).

### CAR Hinge Domains

The extracellular domain of the CARs of the disclosure can comprise a "hinge" domain (or hinge region). The term generally to any polypeptide that functions to link the transmembrane domain in a CAR to the extracellular antigen binding domain in a CAR. In particular, hinge domains can be used to provide more flexibility and accessibility for the extracellular antigen binding domain.

A hinge domain can comprise up to 300 amino acids-in some embodiments 10 to 100 amino acids or in some embodiments 25 to 50 amino acids. The hinge domain can be derived from all or part of naturally occurring molecules, such as from all or part of the extracellular region of CD8, CD4, CD28, 4-1BB, or IgG (in particular, the hinge region of an IgG; it will be appreciated that the hinge region may contain some or all of a member of the immunoglobulin family such as IgG1, IgG2, IgG3, IgG4, IgA, IgD, IgE, IgM, or fragment thereof), or from all or part of an antibody heavy-chain constant region. Alternatively, the hinge domain may be a synthetic sequence that corresponds to a naturally occurring sequence, or may be an entirely synthetic hinge sequence. In some embodiments said hinge domain is a part of human CD8α chain (e.g., NP_001139345.1). In another particular embodiment, said hinge and transmembrane domains comprise a part of human CD8α chain. In some embodiments, the hinge domain of CARs described herein comprises a subsequence of CD8α, an IgG1, IgG4, PD-1 or an FcγRIIIα, in particular the hinge region of any of an CD8α, an IgG1, IgG4, PD-1 or an FcγRIIIα. In some embodiments, the hinge domain comprises a human CD8α hinge, a human IgG1 hinge, a human IgG4, a human PD-1 or a human FcγRIIIα hinge. In some embodiments the CARs disclosed herein comprise a scFv, CD8α human hinge and transmembrane domains, the CD3ζ signaling domain, and 4-1BB signaling domain.

### CAR Transmembrane Domains

The CARs provided herein are designed with a transmembrane domain that is fused to the extracellular domain of the CAR. It can similarly be fused to the intracellular domain of the CAR. In some instances, the transmembrane domain can be selected or modified by amino acid substitution to avoid binding of such domains to the transmembrane domains of the same or different surface membrane proteins to minimize interactions with other members of the receptor complex. In some embodiments, short linkers can form linkages between any or some of the extracellular, transmembrane, and intracellular domains of the CAR.

Transmembrane regions can be synthetic (not-naturally-occurring) or can be derived from (comprise, or correspond to a fragment of) CD28, OX-40, 4-1BB/CD137, CD2, CD7, CD27, CD30, CD40, programmed death-1 (PD-1), inducible T cell costimulator (ICOS), lymphocyte function-associated antigen-1 (LFA-1, CD1-1a/CD18), CD3 gamma, CD3 delta, CD3 epsilon, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptors, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CD1 1d, ITGAE, CD103, ITGAL, CD1 1a, LFA-1, ITGAM, CD1 1b, ITGAX, CD1 1c, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, or any combination thereof.

In some embodiments, the transmembrane domain in the CAR of the disclosure is a CD8α transmembrane domain.

In some embodiments, the transmembrane domain in the CAR of the disclosure is a CD28 transmembrane domain.

### CAR Intracellular Domains

The intracellular (cytoplasmic) domain of a CAR can provide activation of at least one of the normal effector functions of the immune cell comprising the CAR. Effector function of a T cell, for example, can refer to cytolytic activity or helper activity, including the secretion of cytokines.

It will be appreciated that suitable (e.g., activating) intracellular domains include, but are not limited to signaling domains derived from (comprise, or correspond to all or a fragment of) CD28, OX-40, 4-1BB/CD137, CD2, CD7, CD27, CD30, CD40, programmed death-1 (PD-1), inducible T cell costimulator (ICOS), lymphocyte function-associated antigen-1 (LFA-1, CD1-1a/CD18), CD3 gamma, CD3 delta, CD3 epsilon, CD247, CD276 (B7-H3), LIGHT, (TNFSF14), NKG2C, Ig alpha (CD79a), DAP-10, Fc gamma receptor, MHC class 1 molecule, TNF receptor proteins, an Immunoglobulin protein, cytokine receptor, integrins, Signaling Lymphocytic Activation Molecules (SLAM proteins), activating NK cell receptors, BTLA, a Toll ligand receptors, ICAM-1, B7-H3, CDS, ICAM-1, GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL-2R beta, IL-2R gamma, IL-7R alpha, ITGA4, VLA1, CD49a, ITGA4, IA4, CD49D, ITGA6, VLA-6, CD49f, ITGAD, CD1 1d, ITGAE, CD103, ITGAL, CD1 1a, LFA-1, ITGAM, CD1 1b, ITGAX, CD1 1c, ITGB1, CD29, ITGB2, CD18, LFA-1, ITGB7, NKG2D, TNFR2, TRANCE/RANKL, DNAM1 (CD226), SLAMF4 (CD244, 2B4), CD84, CD96 (Tactile), CEACAM1, CRT AM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, a ligand that specifically binds with CD83, or any combination thereof.

In some embodiments, the intracellular/cytoplasmic domain of a CAR can be designed to comprise the 41BB/CD137 domain by itself or combined with any other desired intracellular domain(s) useful in the context of a CAR. The complete native amino acid sequence of 41BB/CD137 is described in NCBI Reference Sequence: NP_001552.2. The complete native 41BB/CD137 nucleic acid sequence is described in NCBI Reference Sequence: NM_001561.5.

In some embodiments, the intracellular/cytoplasmic domain of the CAR can be designed to comprise a CD28 domain by itself or combined with any other desired intracellular domain(s) useful in the context of a CAR of the instant disclosure. The complete native amino acid sequence of CD28 is described in NCBI Reference Sequence: NP_006130.1. The complete native CD28 nucleic acid sequence is described in NCBI Reference Sequence: NM_006139.1.

In some embodiments, the intracellular/cytoplasmic domain of a CAR can be designed to comprise all or a fragment of the CD3 zeta domain by itself or combined with any other desired intracellular domain(s) useful in the context of a CAR.

In some embodiments the intracellular signaling domain of a CAR comprises a domain of a co-stimulatory molecule. In some embodiments, the intracellular signaling domain of a CAR comprises a part of co-stimulatory molecule selected from the group consisting of fragment of 41BB (GenBank: AAA53133.) and CD28 (NP_006130.1).

### Engineered Immune Cells Comprising CARs

Provided herein are engineered immune cells and populations of engineered immune cells expressing CAR (e.g., CAR-T cells or CAR+ cells), which are depleted of cells having expressing endogenous TCR.

In some embodiments, an engineered immune cell comprises a CAR T cell, each CAR T cell comprising an extracellular antigen-binding domain and has reduced or eliminated expression of endogenous TCR. In some embodiments, a population of engineered immune cells comprises a population of CAR T cells, each CAR T cell comprising two or more different extracellular antigen-binding domain and has reduced or eliminated expression of endogenous TCR. In some embodiments, an immune cell comprises a population of CARs, each CAR T cell comprising the same extracellular antigen-binding domains and has reduced or eliminated expression of endogenous TCR.

The engineered immune cells can be allogeneic or autologous.

In some embodiments, an engineered immune cell or population of engineered immune cells is a T cell (e.g., inflammatory T-lymphocyte cytotoxic T-lymphocyte, regulatory T-lymphocyte, helper T-lymphocyte, tumor infiltrating lymphocyte (TIL)), NK cell, NK-T-cell, TCR-expressing cell, dendritic cell, killer dendritic cell, a mast cell, or a B-cell, and expresses a CAR. In some embodiments, the T cell can be derived from the group consisting of CD4+ T lymphocytes, CD8+ T lymphocytes or population comprising a combination of CD4+ and CD8+ T cells.

In some embodiments, an engineered immune cell or population of engineered immune cells that are generated using the disclosed methods can be derived from, for example without limitation, a stem cell. The stem cells can be adult stem cells, non-human embryonic stem cells, more particularly non-human stem cells, cord blood stem cells, progenitor cells, bone marrow stem cells, induced pluripotent stem cells, totipotent stem cells or hematopoietic stem cells.

In some embodiments, an engineered immune cell or a population of immune cells that are generated using the disclosed methods is obtained or prepared from peripheral blood. In some embodiments, an engineered immune cell is obtained or prepared from peripheral blood mononuclear cells (PBMCs). In some embodiments, an engineered immune cell is obtained or prepared from bone marrow. In some embodiments, an engineered immune cell is obtained or prepared from umbilical cord blood. In some embodiments, the cell is a human cell. In some embodiments, the cell is transfected or transduced by the nucleic acid vector using a method selected from the group consisting of electroporation, sonoporation, biolistics (e.g., Gene Gun), lipid transfection, polymer transfection, nanoparticles, viral transfection (e.g., retrovirus, lentivirus, AAV) or polyplexes.

In some embodiments, the engineered immune cells expressing at their cell surface membrane an antigen-specific CAR comprise a percentage of stem cell memory and central memory cells greater than 10%, 20%, 30%, 40%, 50%, 60%, 70%, 80%, 90%, or 100%.

In some embodiments, the engineered immune cells expressing at their cell surface membrane an antigen-specific CAR of the disclosure comprise a percentage of stem cell memory and central memory cells of about 10% to about 100%, about 10% to about 90%, about 10% to about 80%, about 10% to about 70%, about 10% to about 60%, about 10% to about 50%, about 10% to about 40%, about 10% to about 30%, about 10% to about 20%, about 15% to about 100%, about 15% to about 90%, about 15% to about 80%, about 15% to about 70%, about 15% to about 60%, about 15% to about 50%, about 15% to about 40%, about 15% to about 30%, about 20% to about 100%, about 20% to about 90%, about 20% to about 80%, about 20% to about 70%, about 20% to about 60%, about 20% to about 50%, about 20% to about 40%, about 20% to about 30%, about 30% to about 100%, about 30% to about 90%, about 30% to about 80%, about 30% to about 70%, about 30% to about 60%, about 30% to about 50%, about 30% to about 40%, about 40% to about 100%, about 40% to about 90%, about 40% to about 80%, about 40% to about 70%, about 40% to about 60%, about 40% to about 50%, about 50% to about 100%, about 50% to about 90%, about 50% to about 80%, about 50% to about 70%, about 50% to about 60%, about 60% to about 100%, about 60% to about 90%, about 60% to about 80%, about 60% to about 70%, about 70% to about 90%, about 70% to about 80%, about 80% to about 100%, about 80% to about 90%, about 90% to about 100%, about 25% to about 50%, about 75% to about 100%, or about 50% to about 75%.

In some embodiments, engineered immune cells expressing at their cell surface membrane an antigen-specific CAR comprises a percentage of stem cell memory and central memory cells greater than 10%, 20%, 30%, 40%, 50%, or 60%.

In some embodiments, engineered immune cells expressing at their cell surface membrane an antigen-specific CAR comprise a percentage of stem cell memory and central memory cells of about 10% to about 60%, about 10% to about 50%, about 10% to about 40%, about 15% to about 50%, about 15% to about 40%, about 20% to about 60%, or about 20% to about 70%.

In some embodiments, engineered immune cells expressing at their cell surface membrane an antigen-specific CAR are enriched in T_{CM} and/or T_{SCM} cells such that the engineered immune cells comprise at least about 60%, 65%, 70%, 75%, or 80% combined T_{CM} and T_{SCM} cells. In some embodiments, engineered immune cells expressing at their cell surface membrane an antigen-specific CAR are enriched in T_{CM} and/or T_{SCM} cells such that the engineered immune cells comprise at least about 70% combined T_{CM} and T_{SCM} cells. In some embodiments, engineered immune cells expressing at their cell surface membrane an antigen-specific CAR e enriched in T_{CM} and/or T_{SCM} cells such that the engineered immune cells comprise at least about 75% combined T_{CM} and/or T_{SCM} cells.

In some embodiments, engineered immune cells expressing at their cell surface membrane an antigen-specific CAR of the disclosure comprise are enriched in T_{CM} and/or T_{SCM} cells such that the engineered immune cells comprise at least about 20%, 25%, 30%, 35%, 40%, 45%, 50%, 55%, or 60% T_{SCM} cells. In some embodiments, engineered immune cells expressing at their cell surface membrane an antigen-specific CAR of the disclosure comprise are enriched in T_{CM} and/or T_{SCM} cells such that the engineered immune cells comprise at least about 30%, 35%, 40%, or 45% T_{SCM} cells.

In some embodiments, an engineered immune cell is an inflammatory T-lymphocyte that expresses a CAR. In some embodiments, an engineered immune cell is a cytotoxic T-lymphocyte that expresses a CAR. In some embodiments, an engineered immune cell is a regulatory T-lymphocyte that expresses a CAR. In some embodiments, an engineered immune cell is a helper T-lymphocyte that expresses a CAR.

In some embodiments, an engineered immune cell according to the present disclosure can comprise one or more disrupted or inactivated genes. In some embodiments, a gene for a target antigen (e.g., EGFRvIII, Flt3, WT-1, CD20, CD23, CD30, CD38, CD33, CD133, MHC-WT1, TSPAN10, MHC-PRAME, Liv1, ADAM10, CHRNA2, LeY, NKGD2D, CS1, CD44v6, ROR1, Claudin-18.2, Muc17, FAP alpha, Ly6G6D, c6orf23, G6D, MEGT1, NG25, CD19, BCMA, FLT3, CD70, DLL3, or CD34, CD70) can be knocked out to introduce a CAR targeting the same antigen (e.g., a EGFRvIII, Flt3, WT-1, CD20, CD23, CD30, CD38, CD33, CD133, MHC-WT1, TSPAN10, MHC-PRAME, Liv1, ADAM10, CHRNA2, LeY, NKGD2D, CS1, CD44v6, ROR1, Claudin-18.2, Muc17, FAP alpha, Ly6G6D, c6orf23, G6D, MEGT1, NG25, CD19, BCMA, FLT3, CD70, DLL3, or CD34, CD70 CAR) to avoid induced CAR activation. As described herein, in some embodiments, an engineered immune cell according to the present disclosure comprises one disrupted or inactivated gene selected from the group consisting of MHC1 (β2M), MHC2 (CIITA), EGFRvIII, Flt3, WT-1, CD20, CD23, CD30, CD38, CD33, CD133, MHC-WT1, TSPAN10, MHC-PRAME, Liv1, ADAM10, CHRNA2, LeY, NKGD2D, CS1, CD44v6, ROR1, Claudin-18.2, Muc17, FAP alpha, Ly6G6D, c6orf23, G6D, MEGT1, NG25, CD19, BCMA, FLT3, CD70, DLL3, or CD34, CD70, TCRα and TCRβ and/or expresses a CAR or a multi-chain CAR. In some embodiments, a cell comprises a multi-chain CAR. In some embodiments, the isolated cell comprises two disrupted or inactivated genes selected from the group consisting of: CD52 and TCRα, CDR52 and TCRβ, PD-1 and TCRα, PD-1 and TCRβ, MHC-1 and TCRα, MHC-1 and TCRβ, MHC2 and TCRα, MHC2 and TCRβ and/or expresses a CAR or a multi-chain CAR.

In some embodiments the method comprises disrupting or inactivating one or more genes by introducing into the cells an endonuclease able to selectively inactivate a gene by selective DNA cleavage. In some embodiments the endonuclease can be, for example, a zinc finger nuclease (ZFN), megaTAL nuclease, meganuclease, transcription activator-like effector nuclease (TALE-nuclease, or TALEN^{®}), or CRISPR (e.g., a Cas9 or Cas12) endonuclease.

In some embodiments, a TCR+ cell or population thereof is rendered nonfunctional in the cells by disrupting or inactivating endogenous TCRα gene and/or TCRβ gene(s). The depletion methods of the instant disclosure are particularly useful for removing any remaining TCR+ T cells from a population of cells, following inactivation of the TRCα gene. Engineered immune cells generated using the methods disclosed herein can be used in for treating patients in need thereof by preventing or reducing Host versus Graft (HvGD) rejection and Graft versus Host Disease (GvHD); therefore in the scope of the present disclosure is a method of treating patients in need thereof by preventing or reducing Host versus Graft (HvG) rejection and Graft versus Host Disease (GvHD) comprising treating said patient by administering to said patient an effective amount of engineered immune cells comprising disrupted or inactivated TCRα and/or TCRβ genes.

The present disclosure provides methods of increasing the purity of a population of engineered immune cells lacking or having reduced endogenous TCR expression. In some embodiments, the engineered immune cells comprise less than 1.0% TCR+ cells, less than 0.9% TCR+ cells, less than 0.8% TCR+ cells, less than 0.7% TCR+ cells, less than 0.6% TCR+ cells, less than 0.5% TCR+ cells, less than 0.4% TCR+ cells, less than 0.3% TCR+ cells, less than 0.2% TCR+ cells, or less than 0.1% TCR+ cells. Such a population can be a product of the disclosed methods.

In some embodiments, a population of engineered immune cells comprise less than 0.1% TCR+ cells. In some embodiments, the engineered immune cells of the comprise less than 0.09% TCR+ cells, less than 0.08% TCR+ cells, less than 0.07% TCR+ cells, less than 0.06% TCR+ cells, less than 0.05% TCR+ cells, less than 0.04% TCR+ cells, less than 0.03% TCR+ cells, less than 0.02% TCR+ cells, less than 0.01% TCR+ cells. Such a population can be a product of the disclosed methods.

In some embodiments, a population of engineered immune cells comprise between about 0.01-0.001% TCR+ cells. Such a population can be a product of the disclosed methods.

The disclosure also provides engineered immune cells comprising any of the CARs described herein, and can also feature reduced or eliminated expression of one or more endogenous genes. In some embodiments, a CAR can be introduced into an immune cell as a transgene via a plasmid vector. In some embodiments, the plasmid vector can also contain, for example, a selection marker which provides for identification and/or selection of cells which received the vector.

### Manufacture of TCR- Engineered Immune Cells and Populations of TCR- Engineered Immune Cells (including CAR T cells)

Provided herein are methods of depleting cells expressing an endogenous TCR from a population of immune cells (including engineered immune cells such as CAR+ cells). As described herein, labeling the population of cells with an anti-TCR antibody and an anti-CD3 antibody, separating the anti-TCR and anti-CD3 labeled cells from the unlabeled cells, and collecting the unlabeled cells, facilitates obtaining a population of cells that are depleted of cells expressing an endogenous TCR. In various embodiments the separating can be generally achieved using magnetic beads, acoustic wave particles, membranes (all of which can be conjugated to a moiety that is recognized by the labelled cells), FACS and other known separation methods. Cells generated using this method also form an aspect of the instant disclosure.

Also provided herein are methods of depleting cells expressing an endogenous TCR from a population of immune cells (including engineered immune cells such as CAR+ cells). As described herein, labeling the population of cells with an anti-TCR antibody and an anti-CD3 antibody and an anti-CD52 antibody, separating the anti-TCR, anti-CD3 labelled and anti-CD52 labeled cells from the unlabeled cells, and collecting the unlabeled cells, facilitates obtaining a population of cells that are depleted of cells expressing an endogenous TCR. In various embodiments the separating can be generally achieved using magnetic beads, acoustic wave particles, membranes (all of which can be conjugated to a moiety that is recognized by the labelled cells), FACS and other known separation methods. Cells generated using this method also form an aspect of the instant disclosure.

Prior to the *in vitro* manipulation or genetic modification of the engineered immune cells described herein, the cells can be obtained from a subject. A population of cells expressing a CAR can be derived from an allogenic or autologous process, as described herein, and can be depleted of endogenous TCR as described herein.

### Genetic modification of isolated immune cells

As described herein, immune cells, such as T cells, can be genetically modified prior to performing the methods provided herein using known methods, or the immune cells can be activated and expanded (or differentiated in the case of iPSC or progenitor cells) in vitro prior to being genetically modified. The immune cells are genetically modified to reduce or eliminate expression of endogenous TCR (TRAC). In some embodiments, the immune cells are genetically modified to reduce or eliminate expression of MHC1(β2M), MHC2, PD1, and/or CD52. In some embodiments, expression of two or more endogenous proteins can be reduce or eliminated. For example, expression of TRAC and CD52 can be reduced or eliminated. In another example, expression of TRAC and a protein that is targeted by a transduced CAR can be reduced or eliminated. In some embodiments, the cells are genetically modified using gene editing technology (e.g., CRISPR/Cas9, a zinc finger nuclease (ZFN), a TALEN^{®}, a MegaTAL, a meganuclease) to reduce or eliminate expression of one or more endogenous proteins (e.g., TCRα, MHC1, MHC2, PD1, CD52). In another embodiment, the immune cells, such as T cells, are genetically modified with the chimeric antigen receptors described herein (e.g., transduced with a viral vector comprising one or more nucleotide sequences encoding a CAR, or by using non-viral means) and then are activated and/or expanded *in vitro.*

Certain methods for making the constructs and engineered immune cells of the disclosure are described in PCT application WO2015/120096 (PCT/US15/14520).

The disclosure provides a method of storing genetically engineered cells expressing CARs. This involves cryopreserving the immune cells such that the cells remain viable upon thawing. A fraction of the immune cells expressing the CARs can be cryopreserved by methods known in the art to provide a permanent source of such cells for the future treatment of patients afflicted with a malignancy. When needed, the cryopreserved transformed immune cells can be thawed, grown and expanded for more such cells. As demonstrated by the Examples and Drawings, populations of cells generated using the depletion methods provided herein can be cryopreserved and later thawed for therapeutic applications.

### Allogeneic CAR T cells

A process for manufacturing allogeneic CAR T therapy, or AlloCARs^{™}, involves harvesting healthy, selected, screened and tested T cells from healthy donors. Next, the T cells are engineered to express CARs, which recognize certain cell surface proteins (e.g., target antigens such as EGFRvIII, WT-1, CD20, CD23, CD30, CD38, CD33, CD133, MHC-WT1, TSPAN10, MHC-PRAME, Liv1, ADAM10, CHRNA2, LeY, NKGD2D, CS1, CD44v6, ROR1, Claudin-18.2, Muc17, FAP alpha, Ly6G6D, c6orf23, G6D, MEGT1, NG25, CD19, BCMA, FLT3, CD70, DLL3, CD52 or CD34) that are expressed in hematologic or solid tumors. Allogeneic T cells are gene edited using gene editing tools such as TALEN^{®}, zinc finger, CRISPR or other gene editing technologies in order to reduce the risk of graft versus host disease (GvHD) and to prevent allogeneic rejection (HvGD) when given to a patient different than the donor.

Expression of an endogenous T cell receptor gene (e.g., TCRα, TCRβ) can be reduced or eliminated in order to avoid GvHD. Expression of endogenous MHC1 (β2M), MHC2 and/or PD1 can also be reduced or eliminated in order to avoid HvGD preventing host immune cells from recognizing MHC1 and MHC2 on graft cells as foreign antigens. Endogenous expression CD52 (cluster of differentiation 52) gene can also be reduced or eliminated in order to render the CAR T product resistant to anti-CD52 antibody treatment. Anti-CD52 antibody treatment lymphodepletion can therefore be used to suppress the host immune system allowing the CAR T to stay engrafted to achieve their full therapeutic impact. As described herein, an engineered immune cell or population thereof for use in allogeneic therapy can comprise multiple knock outs, at least for the purposes exemplified above.

In some embodiments, endogenous expression of the gene encoding programmed cell death protein 1 (PD1), also known as, CD279 can be reduced or eliminated in order to enhance anti-tumor potency.

### Autologous CAR T cells

Autologous chimeric antigen receptor (CAR) T cell therapy, involves collecting a patient's own cells (e.g., white blood cells, including T cells) and genetically engineering the T cells to express CARs that recognize target antigens expressed on the cell surface of one or more specific cancer cells and kill cancer cells. The engineered cells are then cryopreserved and subsequently administered to the patient.

### Pharmaceutical Compositions and Therapy

Pharmaceutical compositions comprising a population of cells prepared using the disclosed methods can be useful for treating a patient having a cancer. Desired treatment amounts of engineered cells of a population generated using the methods provided herein is generally at least 2 cells (for example, at least 1 CD8+ central memory T cell or at least 1 CD4+ helper T cell subset or one of each of a CD8+ and a CD4+ cell) or is more typically greater than 10² cells, and up to 10⁶, up to and including 10⁸ or 10⁹ cells and can be more than 10¹⁰ cells. The number of cells will depend upon the desired use for which the composition is intended, and the type of cells included therein. The density of the desired cells is typically greater than 10⁶ cells/ml and generally is greater than 10⁷ cells/ml, generally 108 cells/ml or greater. The clinically relevant number of immune cells can be apportioned into multiple infusions that cumulatively equal or exceed 10⁵, 10⁶, 10⁷, 10⁸, 10⁹, 10¹⁰, 10¹¹, or 10¹² cells. In some applications of a population of cells generated using the methods of the instant disclosure, particularly since all the infused cells will be redirected to a particular target antigen, lower numbers of cells, in the range of 10⁶/kilogram (10⁶-10¹¹ per patient) may be administered. CAR treatments can be administered multiple times at dosages within these ranges. The cells can be autologous, allogeneic, or heterologous to the patient undergoing therapy.

As described herein, a population of engineered immune cells can be depleted of cells expressing endogenous TCR (e.g., cells are TCR- and/or comprises residual levels of TCR+ cells). In some embodiments, the engineered immune cells comprise less than 1.0% TCR+ cells, less than 0.9% TCR+ cells, less than 0.8% TCR+ cells, less than 0.7% TCR+ cells, less than 0.6% TCR+ cells, less than 0.5% TCR+ cells, less than 0.4% TCR+ cells , less than 0.3% TCR+ cells, less than 0.2% TCR+ cells, or less than 0.1% TCR+ cells. Cell populations having the levels of TCR+ cells recited above are achievable using the methods disclosed herein.

In some embodiments, a population of engineered immune cells comprises less than 0.09% TCR+ cells, less than 0.08% TCR+ cells, less than 0.07% TCR+ cells, less than 0.06% TCR+ cells, less than 0.05% TCR+ cells, less than 0.04% TCR+ cells, less than 0.03% TCR+ cells, less than 0.02% TCR+ cells, less than 0.01% TCR+ cells Cell populations having the levels of TCR+ cells recited above are achievable using the methods disclosed herein.

In some embodiments, a population of engineered immune cells comprises between about 0.01-0.001% TCR+ cells. Cell populations having the levels of TCR+ cells recited above are achievable using the methods disclosed herein.

In some embodiments, a population of engineered immune cells comprises undetectable levels of TCR+ cells. Cell populations having the levels of TCR+ cells recited above are achievable using the methods disclosed herein.

In some embodiments, the population of engineered immune cells comprises greater than 99% TCR- cells, greater than 99.9% TCR- cells, greater than 99.91% TCR-cells, greater than 99.92% TCR- cells, greater than 99.93% TCR- cells greater than 99.94% TCR- cells, greater than 99.95% TCR- cells, greater than 99.96% TCR- cells, greater than 99.97% TCR- cells, or greater than 99.98% TCR- cells. Cell populations having the levels of TCR- cells recited above are achievable using the methods disclosed herein.

In some embodiments, the population of engineered immune cells comprises between about 99.99-99.999% TCR- cells. Cell populations having these levels of TCR-cells recited above are achievable using the methods disclosed herein.

The TCR-depleted CAR-expressing cell populations generated using the methods of the present disclosure can be administered either alone, or in combination with other components such as IL-2 or other cytokines or cell populations. Pharmaceutical compositions of the present disclosure can comprise a CAR-expressing TCR- cell population, such as engineered T cells, as described herein. Compositions of the present disclosure are preferably formulated for infusion or intravenous administration.

### Methods of Treatment

The instant disclosure provides methods for treating or preventing a disease (e.g., cancer) in a patient, comprising administering to a patient in need thereof an effective amount of a population of engineered immune cells (e.g., cells obtained not from the patient, but from a heathy donor) comprising a CAR, wherein the population of engineered immune cells is depleted of cells expressing endogenous TCR (e.g., cells are TCR- and/or comprise residual levels of TCR+ cells).

In some reference examples, the population of engineered immune cells comprises less than 1.0% TCR+ cells, less than 0.9% TCR+ cells, less than 0.8% TCR+ cells, less than 0.7% TCR+ cells, less than 0.6% TCR+ cells, less than 0.5% TCR+ cells, less than 0.4% TCR+ cells , less than 0.3% TCR+ cells, less than 0.2% TCR+ cells, or less than 0.1% TCR+ cells. Cell populations having the levels of TCR+ cells recited above are achievable using the methods disclosed herein.

In some reference examples, the population of engineered immune cells of the comprises less than 0.09% TCR+ cells, less than 0.08% TCR+ cells, less than 0.07% TCR+ cells, less than 0.06% TCR+ cells, less than 0.05% TCR+ cells, less than 0.04% TCR+ cells, less than 0.03% TCR+ cells, less than 0.02% TCR+ cells, less than 0.01% TCR+ cells. Cell populations having the levels of TCR+ cells recited above are achievable using the methods disclosed herein.

In some reference examples, the population of engineered immune cells comprises between about 0.01-0.001% TCR+ cells. In some reference examples, the engineered immune cells comprise undetectable levels of TCR+ cells. Cell populations having these levels of TCR+cells recited above are achievable using the methods disclosed herein.

In some reference examples, the population of engineered immune cells comprises greater than 99% TCR- cells, greater than 99.9% TCR- cells, greater than 99.91% TCR- cells, greater than 99.92% TCR- cells, greater than 99.93% TCR- cells greater than 99.94% TCR- cells, greater than 99.95% TCR- cells, greater than 99.96% TCR- cells, greater than 99.97% TCR- cells, or greater than 99.98% TCR- cells. Cell populations having these levels of TCR+cells recited above are achievable using the methods disclosed herein.

In some reference examples, the population of engineered immune cells comprises between about 99.99-99.999% TCR- cells. Cell populations having these levels of TCR+cells recited above are achievable using the methods disclosed herein.

For reference, methods are provided herein for treating diseases or disorders, including cancer. The methods reduce the likelihood that a patient will be faced with GvHD when treated with an allogneic therapy. In some examples, the disclosure relates administering an effective amount of a population of TCR depleted engineered immune cells, prepared using the methods of the present disclosure, to the subject in need thereof. In some examples, the T cell-mediated immune response is directed against a target cell or cells expressing a cancer antigen. In some examples, the TCR depleted engineered immune cell population comprises cells expressing a chimeric antigen receptor (CAR). In some examples, the target cell is a solid or hematologic tumor cell. In some aspects, the disclosure comprises a method for treating or preventing a malignancy, said method comprising administering to a subject in need thereof an effective amount of a population of TCR depleted engineered immune cells prepared using the methods described herein. In some aspects, the disclosure comprises a method for treating or preventing a malignancy, said method comprising administering to a subject in need thereof an effective amount of a population of TCR depleted engineered immune cells prepared using the methods provided herein, wherein the population of TCR depleted immune cell comprises at least one chimeric antigen receptor, and/or isolated antigen binding domain as described herein. In some examples, a population of TCR depleted CAR-containing immune cells prepared using the methods of the disclosure can be used to treat hematologic malignancies or solid tumors.

In some examples, a population of TCR depleted CAR-containing immune cells prepared using the methods of the instant disclosure can be used to treat small cell lung cancer, melanoma, low grade gliomas, glioblastoma, medullary thyroid cancer, carcinoids, dispersed neuroendocrine tumors in the pancreas, bladder and prostate, testicular cancer, and lung adenocarcinomas with neuroendocrine features. In some examples, a population of TCR depleted the CAR-containing immune cells, e.g., CAR-T cells prepared using the methods of the instant disclosure are used to treat solid tumor cancers. In some examples, a population of TCR depleted CAR-containing immune cells, e.g., CAR-T cells prepared using the methods of the instant disclosure are used to treat Non-Hodgkin lymphoma (NHL), renal cell carcinoma (RCC), Acute lymphocytic leukemia (ALL), multiple myeloma (MM), or acute myeloid leukemia (AML).

Also provided, for reference, are methods for reducing the size of a tumor in a subject, comprising administering to the subject a population of TCR depleted engineered cells prepared using the methods of the instant disclosure to the subject, wherein the cell comprises a chimeric antigen receptor comprising an antigen binding domain which binds to an antigen on the tumor.

In some examples, the subject has a solid tumor, or a blood malignancy such as lymphoma or leukemia (a hematologic cancer). In some examples, a population of TCR depleted engineered cells prepared using the methods of the instant disclosure is delivered to a tumor bed. In some examples, the cancer is present in the bone marrow of the subject and a population of cells prepared using the methods of the instant disclosure is delivered thereto. In some examples the cancer is present in the patient's immune or blood cells and a population of cells prepared using the methods of the instant disclosure is delivered thereto. In some examples, the engineered cells are autologous immune cells, e.g., autologous T cells. In some examples, the engineered cells are allogeneic immune cells, e.g., allogeneic T cells, for which the use of the disclosed methods will be of particular benefit.

A "therapeutically effective amount," "effective dose," "effective amount," or "therapeutically effective dosage" of a therapeutic agent, e.g., a population of TCR depleted engineered CAR T cells generated using the methods of the instant disclosure, is any amount that, when used alone or in combination with another therapeutic agent, protects a subject against the onset of a disease or promotes disease regression evidenced by a decrease in severity of disease symptoms, an increase in frequency and duration of disease symptom-free periods, or a prevention of impairment or disability due to the disease affliction. The ability of a therapeutic agent to promote disease regression can be evaluated using a variety of methods known to the skilled practitioner, such as in human subjects during clinical trials, in animal model systems predictive of efficacy in humans, or by assaying the activity of the agent in in vitro assays.

Desired treatment amounts of TCR depleted engineered cells in a composition comprises at least 2 cells (for example, at least one CD8+ central memory T cell and at least one CD4+ helper T cell subset or at least 2 CD4+ cells or at least 2 CD8+ cells) or is more typically greater than 10² cells, and up to 10⁶, up to and including 10⁸ or 10⁹ cells and can be more than 10¹⁰ cells. The number of cells will depend upon the desired use for which the composition is intended, and the type of cells included therein. It is noted that the methods provided herein can be used to generate populations of TCR depleted engineered immune cells that comprise only CD4+ cells, only CD8+ cells or both CD4+ and CD8+ cells. The density of the desired cells is typically greater than 10⁶ cells/ml and generally is greater than 10⁷ cells/ml, generally 10⁸ cells/ml or greater. The clinically relevant number of immune cells can be apportioned into multiple infusions that cumulatively equal or exceed 10⁵, 10⁶, 10⁷, 10⁸, 10⁹, 10¹⁰, 10¹¹, or 10¹² cells. In some aspects of the present disclosure, particularly since all the infused cells will be redirected to a particular target antigen, lower numbers of cells, in the range of 10⁶/kilogram (10⁶-10¹¹ per patient) may be administered. CAR treatments may be administered multiple times at dosages within these ranges. The cells may be autologous, allogeneic, or heterologous to the patient undergoing therapy.

In some examples, the therapeutically effective amount of TCR depleted CAR T cells prepared using the methods of the instant disclosure is about 1 X 10⁵ cells/kg, about 2 X 10⁵ cells/kg, about 3 X 10⁵ cells/kg, about 4 X 10⁵ cells/kg, about 5 X 10⁵ cells/kg, about 6 X 10⁵ cells/kg, about 7 X 10⁵ cells/kg, about 8 X 10⁵ cells/kg, about 9 X 10⁵ cells/kg, 2 X 10⁶ cells/kg, about 3 X 10⁶ cells/kg, about 4 X 10⁶ cells/kg, about 5 X 10⁶ cells/kg, about 6 X 10⁶ cells/kg, about 7 X 10⁶ cells/kg, about 8 X 10⁶ cells/kg, about 9 X 10⁶ cells/kg, about 1 X 10⁷ cells/kg, about 2 X 10⁷ cells/kg, about 3 X 10⁷ cells/kg, about 4 X 10⁷ cells/kg, about 5 X 10⁷ cells/kg, about 6 X 10⁷ cells/kg, about 7 X 10⁷ cells/kg, about 8 X 10⁷ cells/kg, or about 9 X 10⁷ cells/kg.

In some examples, target doses for CAR+/TCR- T cells range from 1×10⁶ to 2×10⁸ cells/kg, for example 2×10⁶ cells/kg. It will be appreciated that doses above and below this range may be appropriate for certain subjects, and appropriate dose levels can be determined by the healthcare provider as needed. Additionally, multiple doses of cells can be provided in accordance with the disclosure.

In some examples, upon administration to a patient, a population of TCR depleted engineered immune cells prepared using the methods of the instant disclosure expressing at their cell surface any one of the antigen-specific CARs described herein can reduce, kill or lyse endogenous antigen-expressing cells of the patient. In one example, a percentage reduction or lysis of antigen-expressing endogenous cells or cells of a cell line expressing an antigen by engineered immune cells expressing any one of an antigen-specific CARs described herein is at least about or greater than 10%, 15%, 20%, 25%, 30%, 35%, 40%, 45%, 50%, 55%, 60%, 65%, 70%, 75%, 80%, 85%, 90%, or 95%. In one example, a percentage reduction or lysis of antigen-expressing endogenous cells or cells of a cell line expressing an antigen by engineered immune cells expressing antigen-specific CARs is about 5% to about 95%, about 10% to about 95%, about 10% to about 90%, about 10% to about 80%, about 10% to about 70%, about 10% to about 60%, about 10% to about 50%, about 10% to about 40%, about 20% to about 90%, about 20% to about 80%, about 20% to about 70%, about 20% to about 60%, about 20% to about 50%, about 25% to about 75%, or about 25% to about 60%. In one example, the endogenous antigen-expressing cells are endogenous antigen-expressing bone marrow cells.

A variety of additional therapeutic agents can be used in conjunction with the compositions described herein. For example, potentially useful additional therapeutic agents include PD-1 inhibitors such as nivolumab (Opdivo^{®}), pembrolizumab (Keytruda^{®}), pembrolizumab, pidilizumab, and atezolizumab; these compounds can be administered before, concurrent with, or subsequent to, administration of a population of TCR depleted engineered immune cells prepared using the methods provided herein.

In some examples, a composition comprising a population of TCR depleted CAR-expressing immune cells prepared using the methods provided herein can be administered before, concurrent with or subsequent to a therapeutic regimen designed to prevent or treat cytokine release syndrome (CRS) or neurotoxicity, such as an anti-IL6 antibody.

In certain examples, the compositions a population of TCR depleted CAR-containing immune cells prepared using the methods provided herein can be administered to a subject in conjunction with a cytokine. Examples of cytokines are lymphokines, monokines, and traditional polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor (HGF); fibroblast growth factor (FGF); prolactin; placental lactogen; mullerian-inhibiting substance; mouse gonadotropin-associated peptide; inhibin; activin; vascular endothelial growth factor; integrin; thrombopoietin (TPO); nerve growth factors (NGFs) such as NGF-beta; platelet-growth factor; transforming growth factors (TGFs) such as TGF-alpha and TGF-beta; insulin-like growth factor-I and -II; erythropoietin (EPO); osteoinductive factors; interferons such as interferon-alpha, beta, and -gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; IL-15, IL-21 a tumor necrosis factor such as TNF-alpha or TNF-beta; and other polypeptide factors including LIF and kit ligand (KL). As used herein, the term cytokine includes proteins from natural sources or from recombinant cell culture, and biologically active equivalents of the native sequence cytokines.

### Kits and Articles of Manufacture

For reference, the present disclosure provides kits comprising TCR depletion reagents comprising one or more of an anti-TCR antibody, an anti-CD3 antibody and an anti-CD52 antibody. The kits can be employed in the depletion methods provided herein.

In one example, a kit provided herein comprises an anti-TCR antibody and an anti-CD3 antibody. In the kit one or both of the anti-TCR and/or anti-CD3 antibodies can optionally be conjugated to biotin. In a specific example both the anti-TCR antibody and the anti-CD3 antibody are conjugated to biotin.

In one example, a kit provided herein comprises an anti-TCR antibody, an anti-CD3 antibody and an anti-CD52 antibody. In the kit one or all of the anti-TCR and/or anti-CD3 and/or anti-CD52 antibodies can optionally be conjugated to biotin. In a specific example the anti-TCR antibody, the anti-CD3 antibody and the anti-CD52 antibody are all conjugated to biotin.

In another aspect, a kit provided herein can further comprise an anti-biotin antibody that is conjugated to a magnetic nanomatrix microbeads, cell-sized beads or other support, such as a membrane, acoustic wave particle or bead, plastic plate or column. The anti-biotin antibody can be provided in a conjugated form or optionally provided as a naked antibody along with materials helpful for attaching the antibody to a magnetic nanomatrix microbeads, cell-sized beads or other support.

In a specific example, a kit provided herein comprises an anti TCR antibody and an anti-CD3 antibody. In the kit one or both of the anti-TCR and/or anti-CD3 antibodies can optionally be conjugated directly to a magnetic bead, membrane, acoustic wave particle, plastic plate or column.

In a specific example, a kit provided herein comprises an anti TCR antibody, an anti-CD3 antibody and anti-CD52 antibody. In the kit one or both of the anti-TCR and/or anti-CD3 and/or anti-CD52 antibodies can optionally be conjugated directly to a magnetic bead, membrane, acoustic wave particle, plastic plate or column.

It is noted they while the use of anti-biotin antibodies forms one embodiment of the methods disclosed herein, other means of capturing biotin-labelled moieties can be employed. For example, streptavidin, avidin and other biotin-binding moieties can be used in lieu of anti-biotin antibodies in the disclosed methods.

The present disclosure also provides articles of manufacture comprising any of the therapeutic compositions and kits described herein. Examples of an article of manufacture include vessels (e.g. sealed vials) containing a therapeutic (e.g., a population of TCR-depleted cells, which can further comprise a CAR, prepared using the methods disclosed herein).

### EXAMPLES

As shown in the following examples, the disclosed combination antibody approach led to a significant TCR+ cell depletion efficiency improvement, which unexpectedly reduced the residual TCR+% level from 0.1-1% to 0.1-0.01% compared with TCR antibody alone (measured on Day 1 post depletion). These examples demonstrate the significant advantages provided by the disclosed depletion methods over current approaches for depleting TCR+ cells. These advantages can provide a benefit to patients receiving allogeneic therapy in the form of decreased likelihood that the patient will exhibit GvHD.

### Example 1. The combination of anti-TCR and CD3 antibodies increases TCR+ cell depletion efficiency

Using CAR engineered immune cells, endogenous TCR and CD52 gene expression was knocked-out by electroporation of TALENs^{®} directed to the TRAC and CD52 genes. TCR and CD52 knocked out cells were then exposed to TCR depletion reagents. Cells were contacted with a primary anti-TCR antibody conjugated to biotin alone or in combination with an anti-CD3 or anti-CD52 antibody as shown in Table 1.

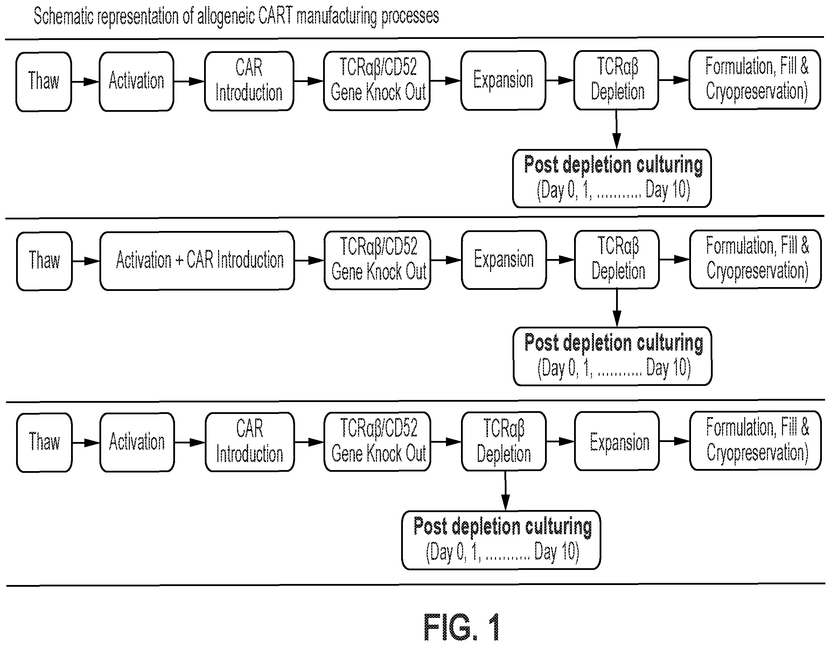

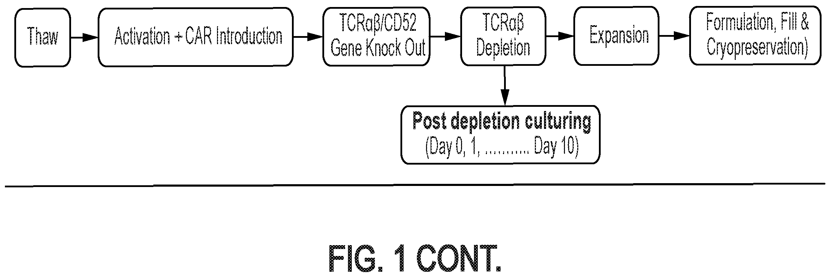

Next, a secondary anti-biotin antibody conjugated with magnetic microbeads (nanoparticles about 50nm in diameter), was further added to the primary antibody-labeled cells in order to bind the magnetic microbeads to any residual TCR+ cells via the primary anti-TCR bioin moeity. The labelled cells were then applied to a magnetic column, using a CliniMACS Prodigy^{®} instrument. TCR+ cells were retained inside the magnetic column, while unlabeled TCR- cells passed through to the product collection bag. The TCR+ cells were later released from column into a waste bag. The TCR+ cell depletion efficiency was explored using the various depletion methods provided in the instant disclosure, and the residual TCR+ cells present in the TCR- cell population fraction were monitored on various days post depletion using anti-TCRα, TCRβ and/or CD3 antibodies. Figure 1 provides a schematic representation of allogenic CART manufacturing processes with different unit operation scenarios.

**Table 1. Experimental design of antibody conditions**

| Conditions | C1 | C2 | C3 | C4 | C5 | C6 | C7 | C8 |
|---|---|---|---|---|---|---|---|---|
| Primary Ab (anti-TCR anti-CD3 anti-CD52 and combinatio ns thereof) and amount | TCR 0.2ul/ millio n cells | TCR+ CD3 (1) 0.2ul+0. 2ul/ million cells | TCR+ CD3 (2) 0.2ul+0. 2ul/ million cells | TCR+ CD3 (1)+ CD52 0.2ul+0. 2ul+0.2 ul/millio n cells | TCR+ CD3 (2)+ CD52 0.2ul+0. 2ul+0.2 ul/millio n cells | TCR+ CD52 0.2ul+0. 2ul/ million cells | 3 x TCR 0.6ul/ million cells | CD52 0.2ul/mi llion cells |
| Secondary (anti-biotin) Ab amount | 0.4ul/ millio n cells | 0.8ul/mi llion cells | 0.8ul/mi llion cells | 1.2ul/mi llion cells | 1.2ul/mi llion cells | 0.8ul/mi llion cells | 1.2ul/mi llion cells | 0.4ul/mi llion cells |

**Table 2. Antibody information**

| **Antibody Target** | **Origin** | **Subtype** | **Clone** | **Conjugation** | **Concentration** |
|---|---|---|---|---|---|
| **Primary** | | | | | |
| TCRαβ GMP | mouse | IgG2b | NA | Biotin | NA |
| CD52 RD | Recombinant human IgG | IgG1 | REA 164 | Biotin | NA |
| CD3 (1) RD | mouse | IgG2ak | BW264/56 | Biotin | NA |
| CD3 (2) RD | Recombinant human IgG | IgG1 | REA613 | Biotin | NA |
| CD3 (3) RD | mouse | IgG2ak | OKT3 | Biotin | 100ug/ml |
| CD3 (4) GMP | mouse | IgG2ak | OKT3 | NA | 1mg/ml |

| **Secondary** | | | | | |
|---|---|---|---|---|---|
| Anti-biotin GMP | mouse | NA | NA | Magnetic microbeads | NA |

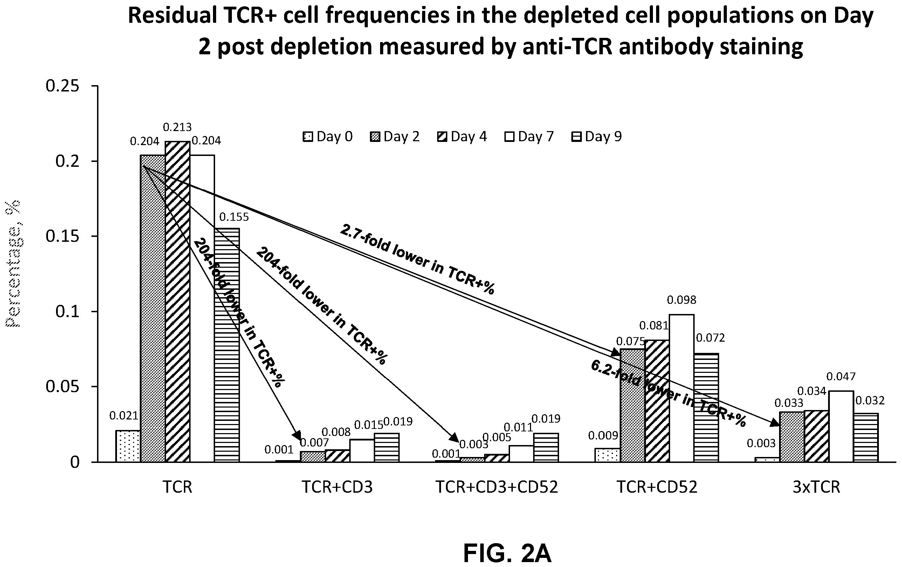

As shown in Figure 2A, the percent of TCR+ cells present in the depleted TCR- product population using various different depletion methods is substantially lower than 1% or less. Figure 2A shows an unexpected decrease of 204-fold in TCR+ cell frequency when anti-TCR and anti-CD3 antibodies were used together in the depletion method, as detected on day 2 post depletion over the control depletion method using low 1x TCR antibody concentration; an equivalent decrease was also observed when the cells were treated with anti-TCR, anti-CD3 and anti-CD52 antibodies. Figure 2A also shows that the use of anti-TCR and anti-CD52 antibodies, as well as a 3x concentration of anti-TCR antibody, was less effective than the anti-TCR and anti-CD3 antibody combination with 3xTCR antibody treatment showing a 6.2-fold decrease and the anti-TCR and anti-CD52 antibody combination showing a 2.7-fold decrease. Interestingly, Figure 2A demonstrates that depletion using anti-CD52 and anti-TCR antibodies was more efficient than using anti-TCR antibody alone. This result was surprising due to the fact that, as noted herein, CD52 has no biological association with TCR or the TCR complex and thus it was unexpected that depletion methods employing these two antibodies together were more efficient at depleting TCR+ cells than just anti-TCR antibodies alone. The results shown in Figure 2A also demonstrate that, unexpectedly, simply increasing the concentration of anti-TCR antibody does not yield markedly better results than using lower concetrations of anti-TCR antibody combined with anti-CD3 antibody.

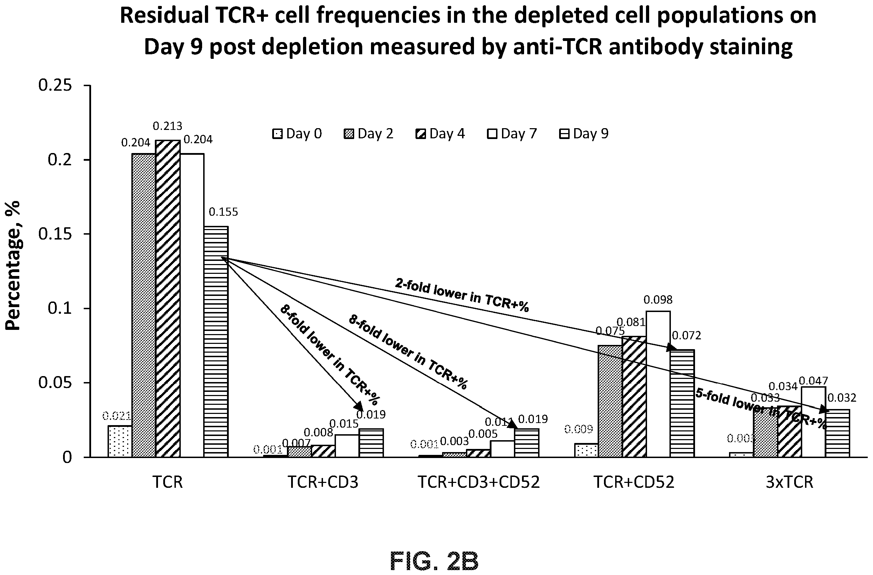

The depleted populations were also studied on day 9 post depletion, and the results are shown in Figure 2B. Figure 2B shows a decrease of 8-fold in TCR+ cell frequency when anti-TCR and anti-CD3 antibodies were used in the depletion method; an equivalent decrease was also observed when the cells were treated with anti-TCR, anti-CD3 and anti-CD52 antibodies. Figure 2B also shows that the use of anti-TCR and anti-CD52 antibodies, as well as a 3x concentration of anti-TCR antibody, was less effective than the anti-TCR and anti-CD3 antibody combination with 3xTCR antibody treatment showing a 5-fold decrease and the anti-TCR and anti-CD52 combination showing a 2-fold decrease. These results demonstrate that simply increasing the concentration of anti-TCR antibodies does not yield markedly better results than using lower concetrations of anti-TCR antibody combined with anti-CD3 antibody.

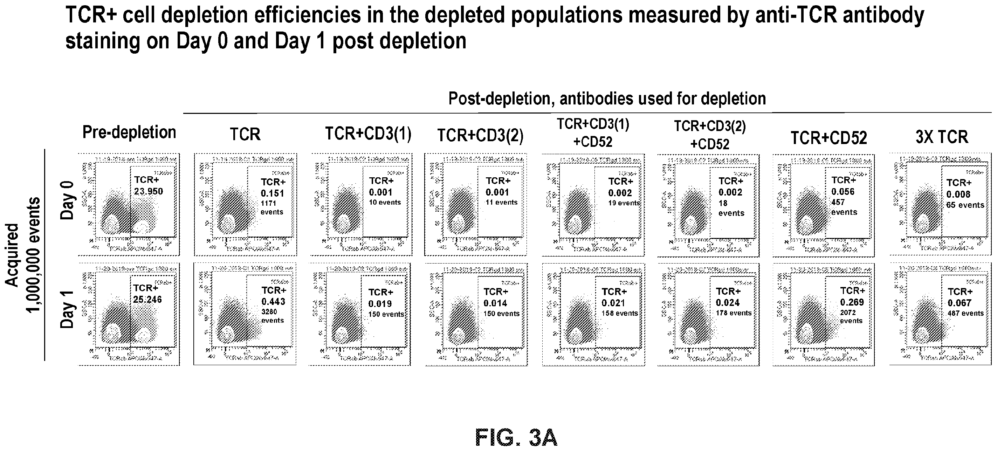

The FACS plots of Figure 3A visualized minimal presence of TCR+ cells when the depletion method was performed using anti-TCR and anti-CD3 antibodies compared with anti-TCR antibodyalone or in combination with anti-CD3 and/or anti-CD52 antibodies, or an increased (3x) concentration of anti-TCR antibody. The data was acquired on Day 0 and Day 1post depletion and compared to the pre-depletion cell samples. The results further demonstrate that simply increasing the concentration of anti-TCR antibodies does not yield markedly better results than using lower concetrations of anti-TCR antibody combined with anti-CD3 antibody over the days studied.

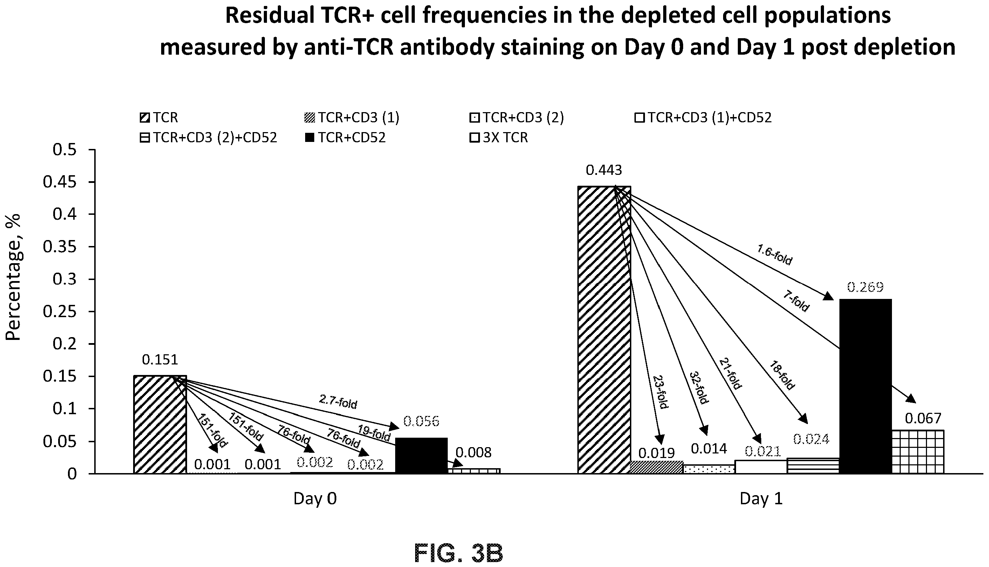

The numerical bar graph of Figure 3B shows a decrease of 151- and 23-32-fold in TCR+ cell frequency when anti-TCR and anti-CD3 antibodies were used in the depletion method on Day 0 and Day 1 post depletion over the control depletion method using low 1x TCR antibody concentration; an equivalent decrease of 70-fold was also observed when the cells were treated with anti-TCR, anti-CD3 and anti-CD52 antibodies. It is noted current TCR depletion operations employ a just a standard 1x TCR antibody concentration, which is a point of differentiation between the disclosed methods and understood to be the current state of the art. Figure 3B also shows that the use of anti-TCR and anti-CD52 antibodies, as well as a 3x concentration of anti-TCR antibody, was less effective than the anti-TCR and anti-CD3 antibody combination with 3xTCR antibody treatment showing a 19- and 7-fold decrease and the anti-TCR and anti-CD52 combination showing a 2.7- and 1.6-fold decrease. These results further demonstrate that simply increasing the concentration of anti-TCR antibodies does not yield markedly better results than using lower concetrations of anti-TCR antibody combined with anti-CD3 antibody over the days studied.

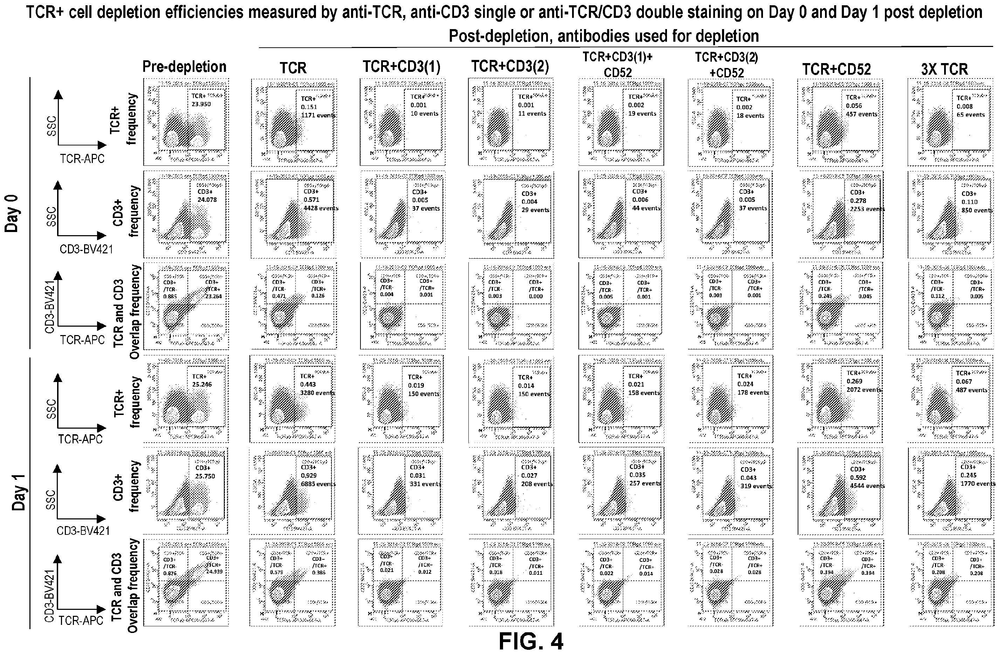

Figure 4 is a FACS plot of data gathered on Day 0 and Day 1 post depletion and further demonstrates that the combination of anti-TCR and anti-CD3 antibodies provides a higher level of TCR depletion than the use of anti-TCR antibody alone at high (3X) or low (1X) concentration, and than the use of anti-TCR antibody in combination with anti-CD52 antibody. The residual TCR+ cells after depletion were visualized by anti-TCR, anti-CD3 single or anti-TCR/CD3 double staining, and they are expected to be stained positively for either TCR+, CD3+ or CD3+/TCR+. The small population of cells stainined as CD3+/TCR-reflects the remaining TCRγδ T cells present in the depleted TCR- cell population since TCRγδ T cells are expected to be removed through anti-CD3 antibody.

### Example 2. Post Depletion Culturing

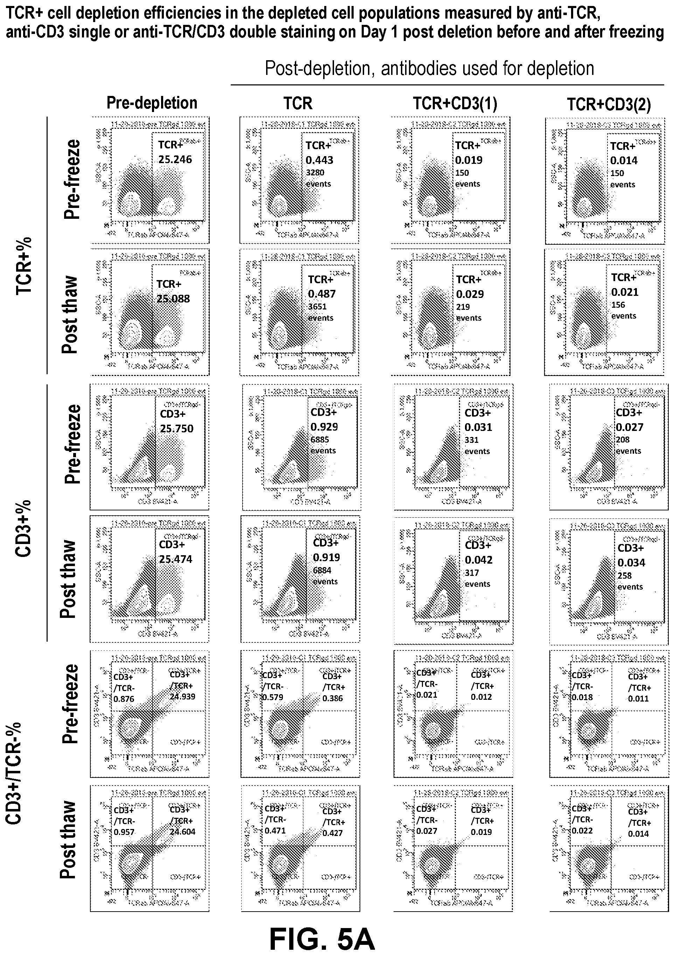

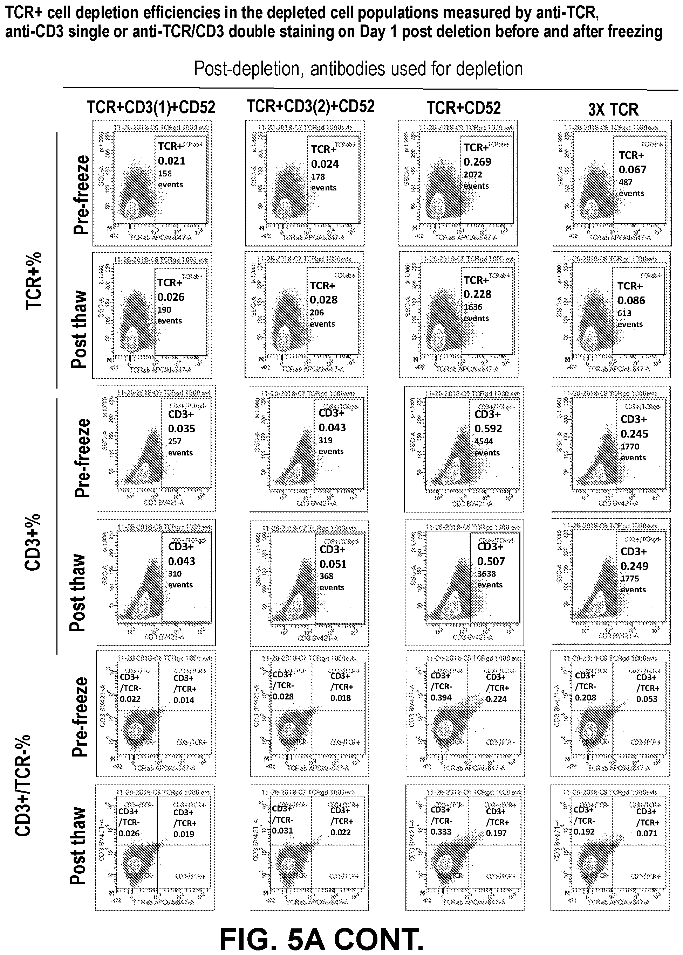

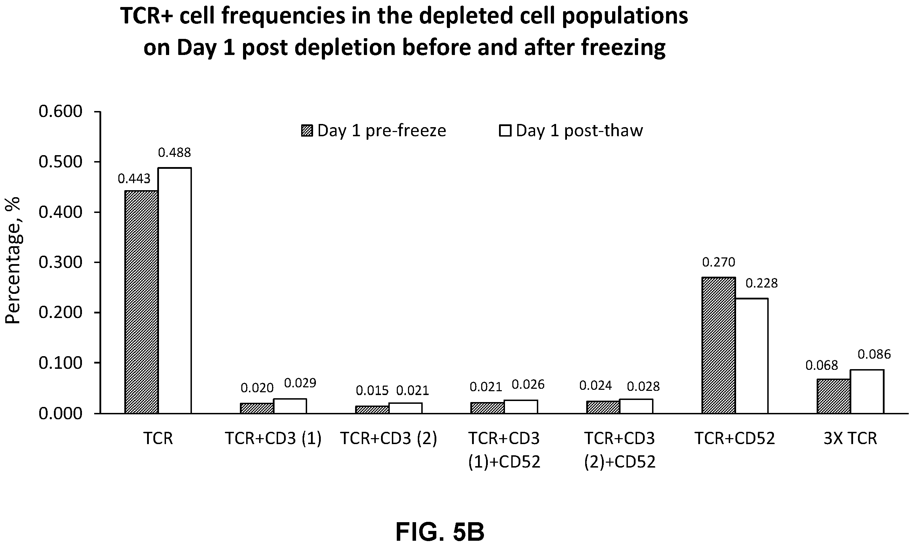

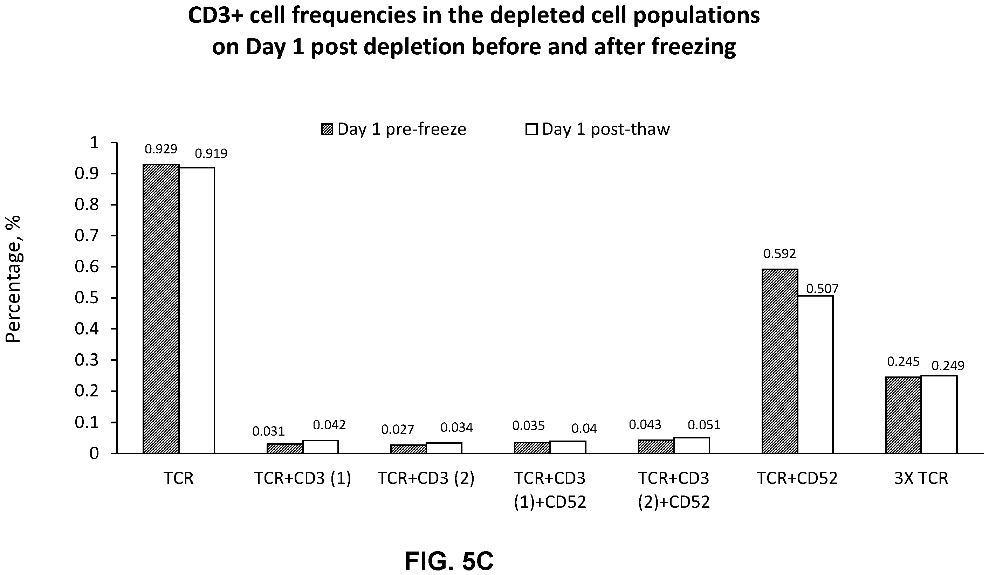

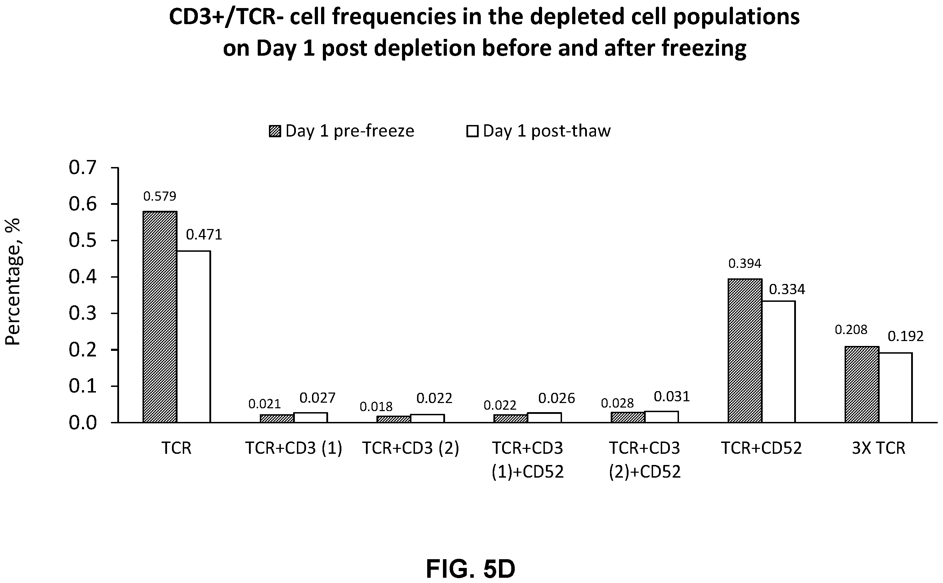

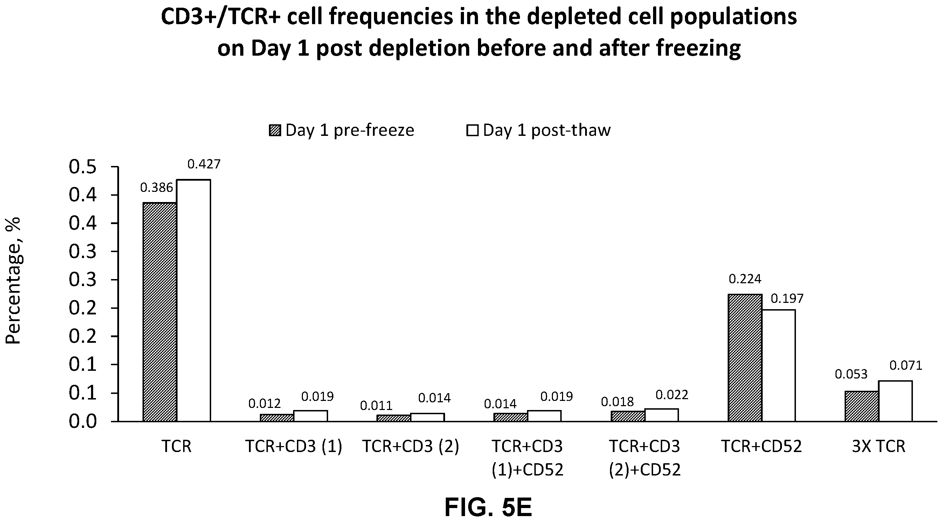

This example demonstrates that populations of depleted immune cells retained the same low residual TCR+ levels during culturing post depletion. On Day 1 post depletion, the depleted cells were frozen and later thawed for post-depletion culturing. TCR and CD3 expression levels were detected before freezing and immediately after thawing. As shown in Figures 5A-5E, there was no significant difference in either TCR and CD3 frequency due to the freeze-thaw cycle; Figure 5A depicts the frequency of TCR+, CD3+, CD3+/TCR- and CD3+/TCR+ cells pre-freezing and post thawing by FACS plot using anti-TCR, anti-CD3 single staining or anti-TCR/CD3 double staining, Figure 5B, depicts the frequency of TCR+ cells pre-freezing and post thawing using anti-TCR antibody in a numerical manner, Figure 5C depicts the frequency of CD3+ cells pre-freezing and post thawing using anti-CD3 antibody in a numerical manner, Figure 5D depicts the frequency of CD3+/TCR- cells pre-freezing and post thawing using anti-TCR/CD3 antibodies in a numerical manner, and Figure 5E depicts the frequency of CD3+/TCR+ cells pre-freezing and post thawing using anti-TCR/CD3 antibodies in a numerical manner.

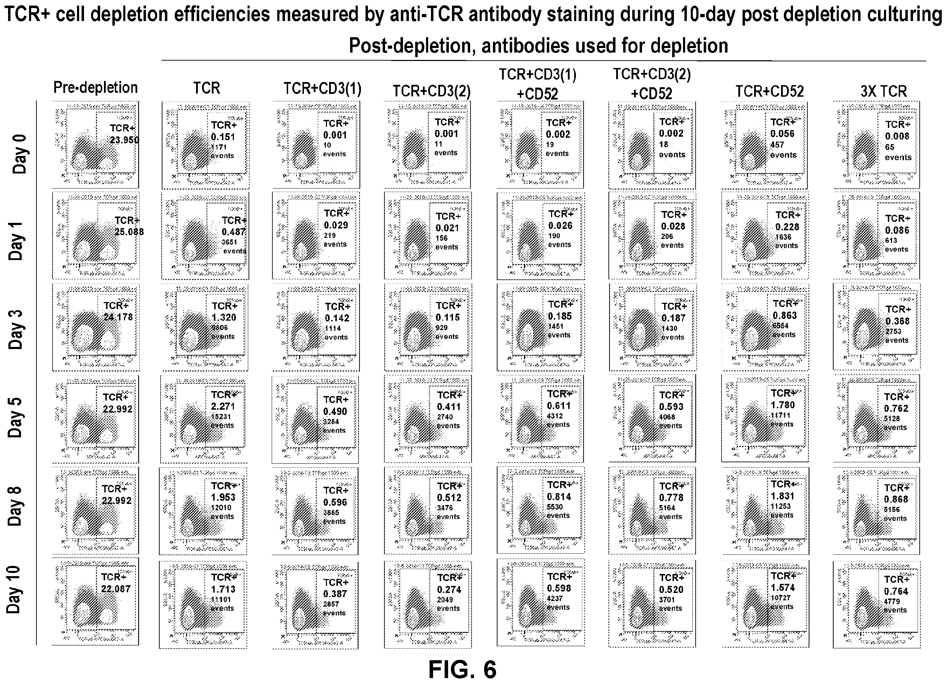

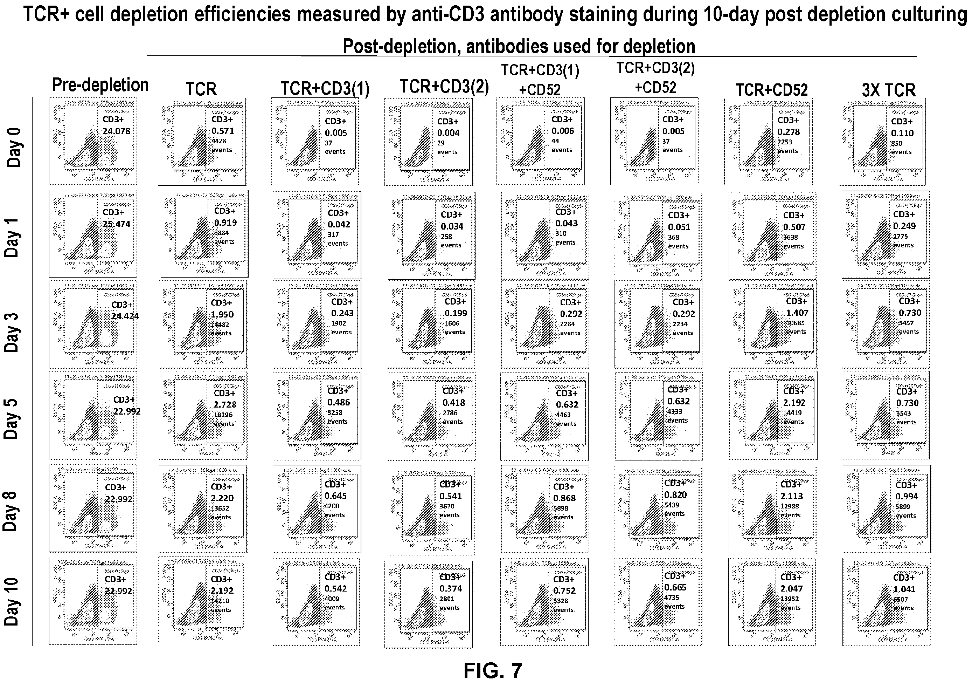

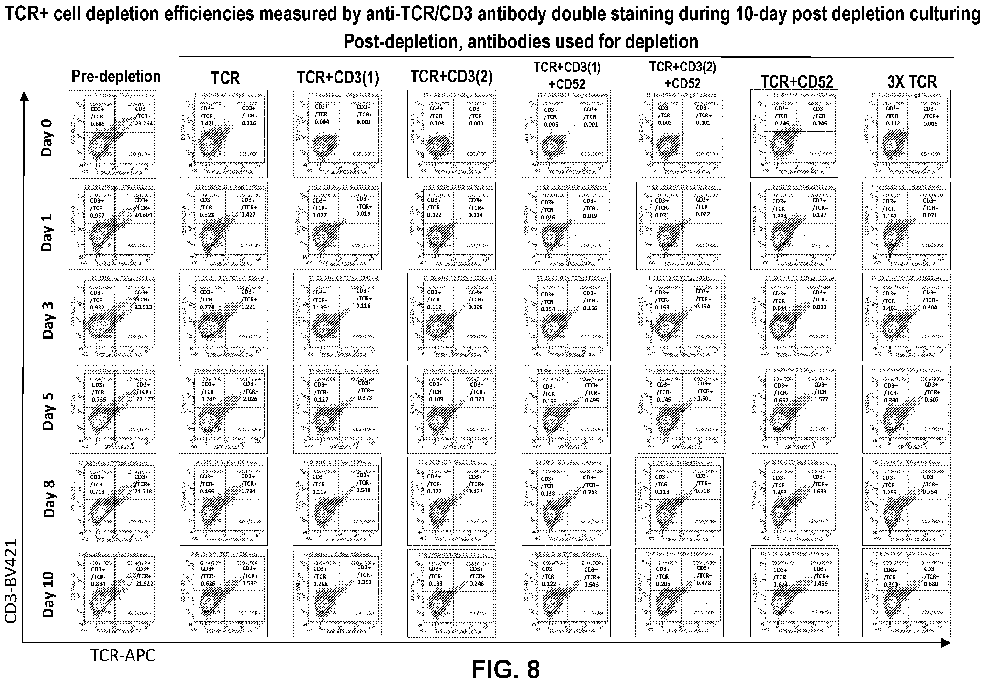

To determine whether the residual TCR+ cell frequency in the depleted TCR- cell populations would increase with the culturing time, the depleted cells were cultured for 10 days after depletion. TCR and CD3 cell frequencies were detected by using anti-TCR (Figure 6) and anti-CD3 (Figure 7) antibody single staining or anti-TCR/CD3 (Figure 8) antibody double staining every 2-3 days during the whole culturing process. Specifically, the FACS plots of Figure 6, 7, and 8 despite a gradual increase trend in TCR+, CD3+, or CD3+/TCR+ cell frequency followed by reaching a peak around 5-7 days during post depletion culturing for all the depletion methods, the cultures depleted using a combination of anti-TCR, anti-CD3 and/or anti-CD52 antibodies maintained the same lower frequency of TCR+ or CD3+ cells over time than those cultures treated with anti-TCR antibody alone at low or high concentration, or anti-TCR antibody in combination with anti-CD52 antibody.

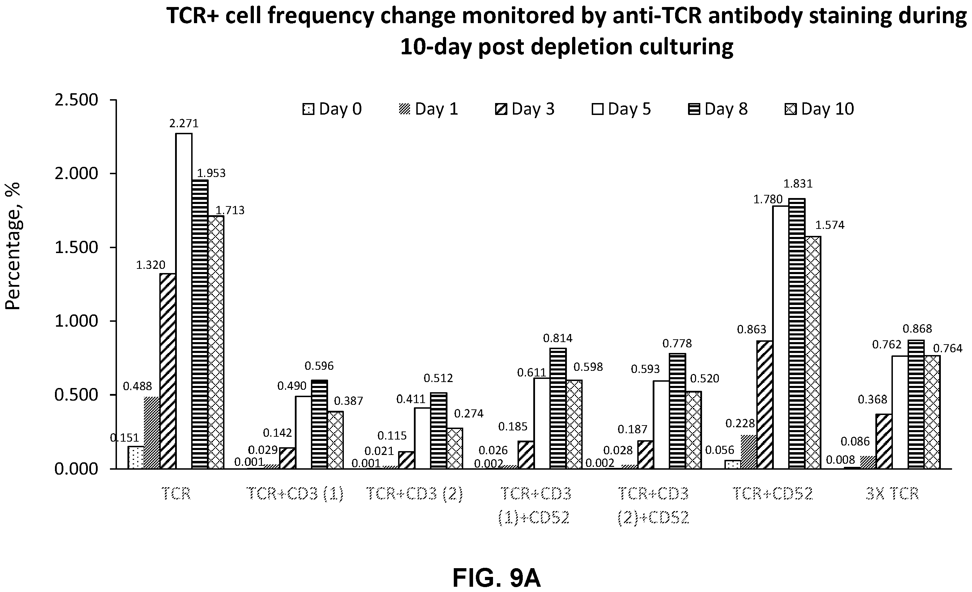

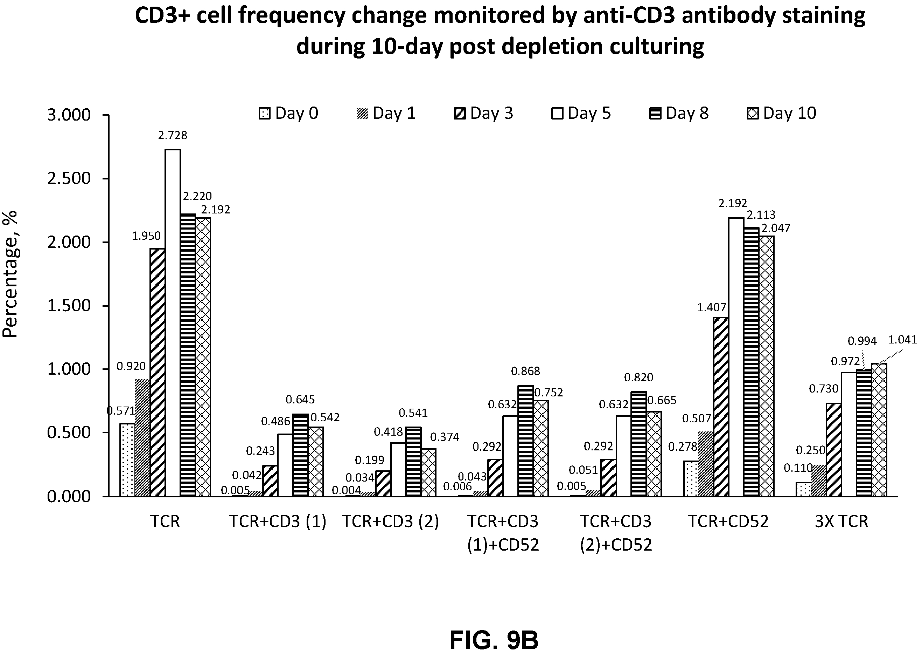

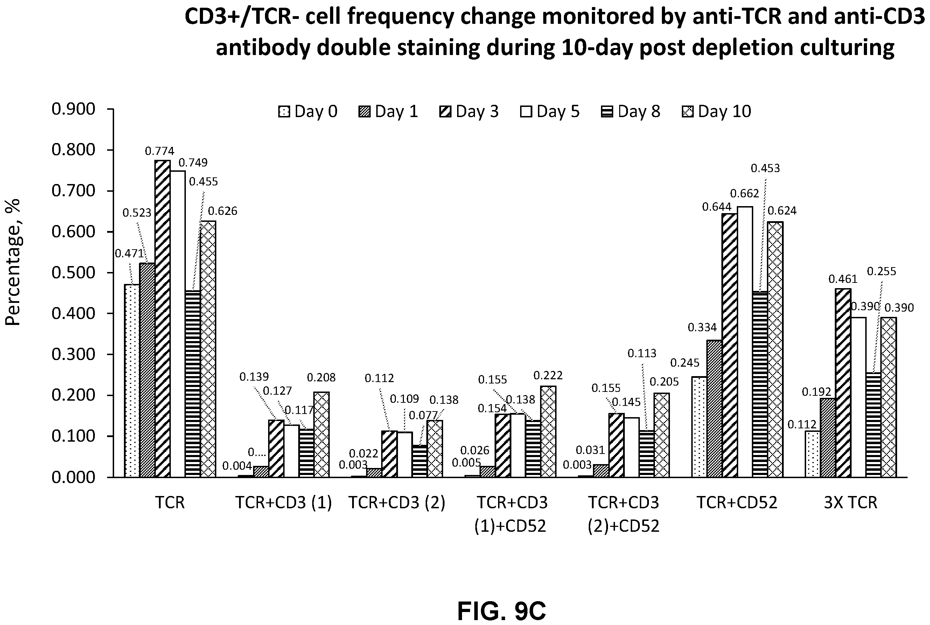

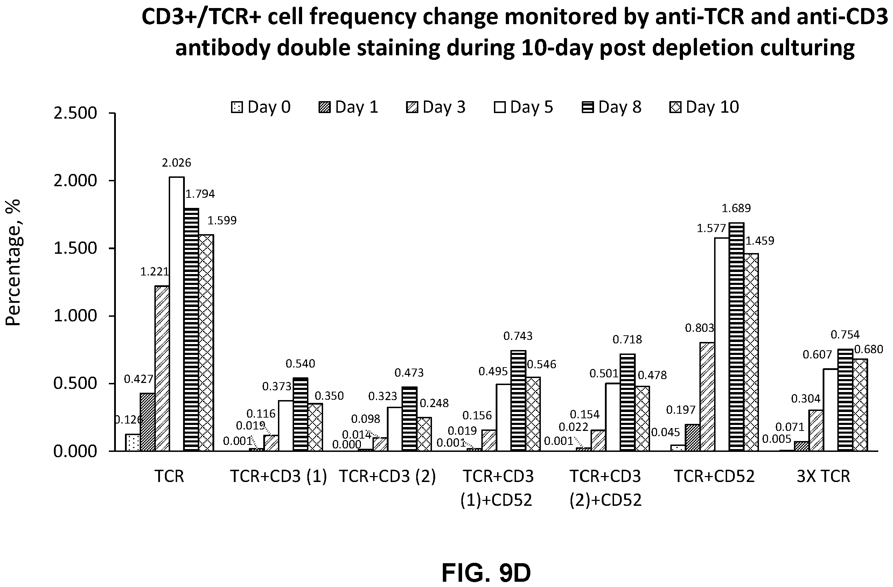

Continuing, the bar graphs of Figure 9A, 9B, 9C and 9D depict TCR+, CD3+, CD3+/TCR- or CD3+/TCR+ frequency during post depletion culturing in a numerical manner.

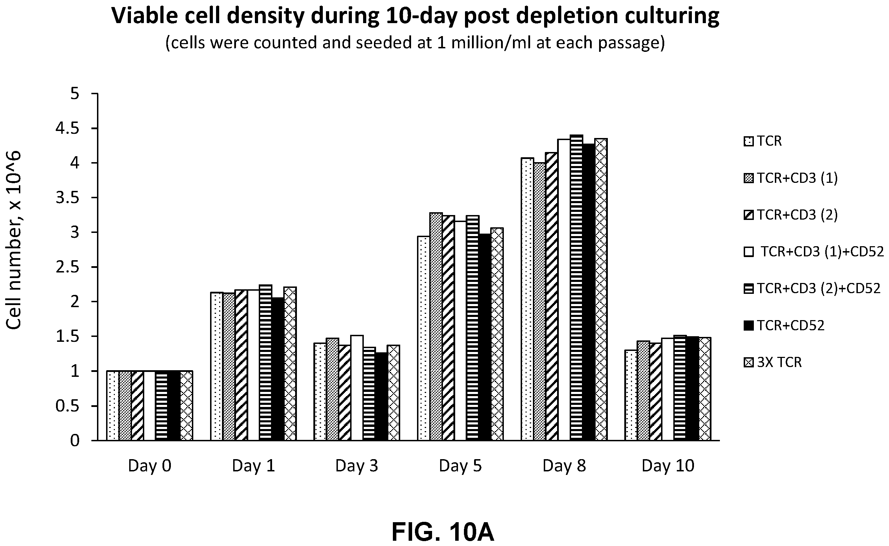

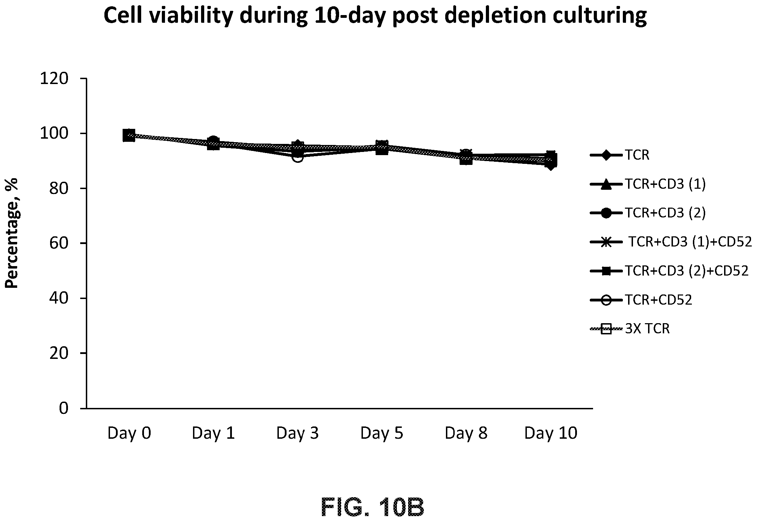

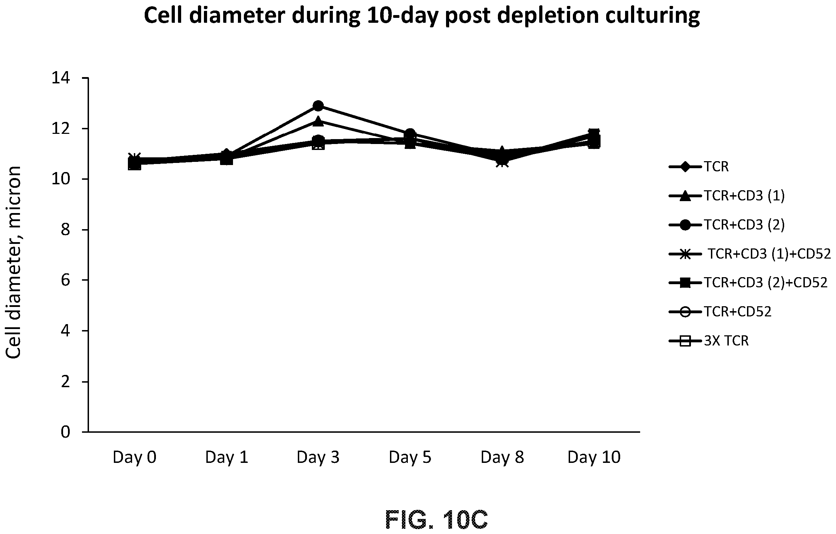

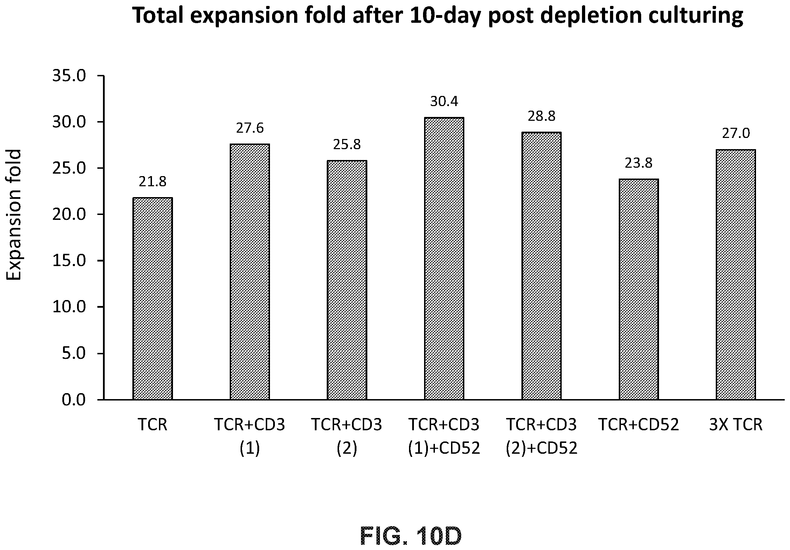

Additionally, the cell growth status, including viable cell density (Figure 10A), viability (Figure 10B), cell diameter (Figure 10C) at each passage and total expansion fold (Figure 10D), were also monitored during post depletion cultutirng period. Overall, comparable cell growth charaterisitics were observed for all the studied depeletion methods.

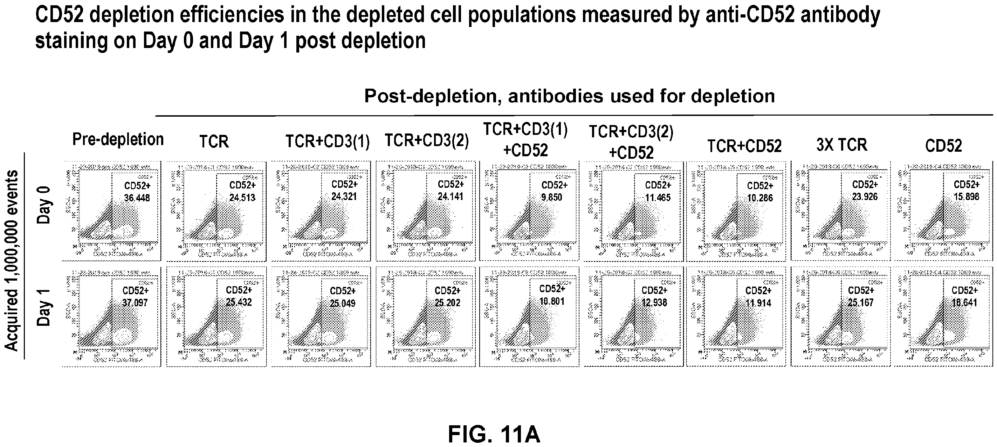

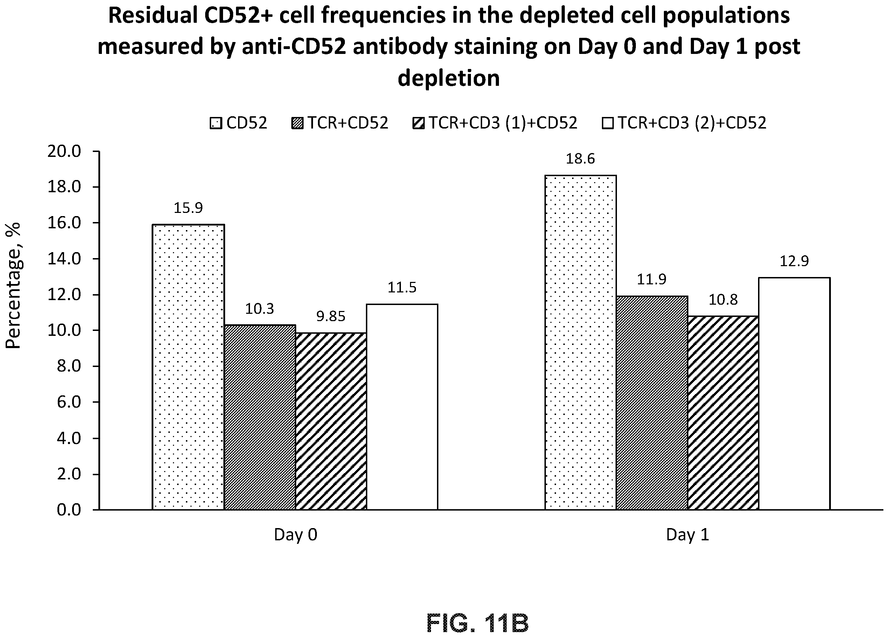

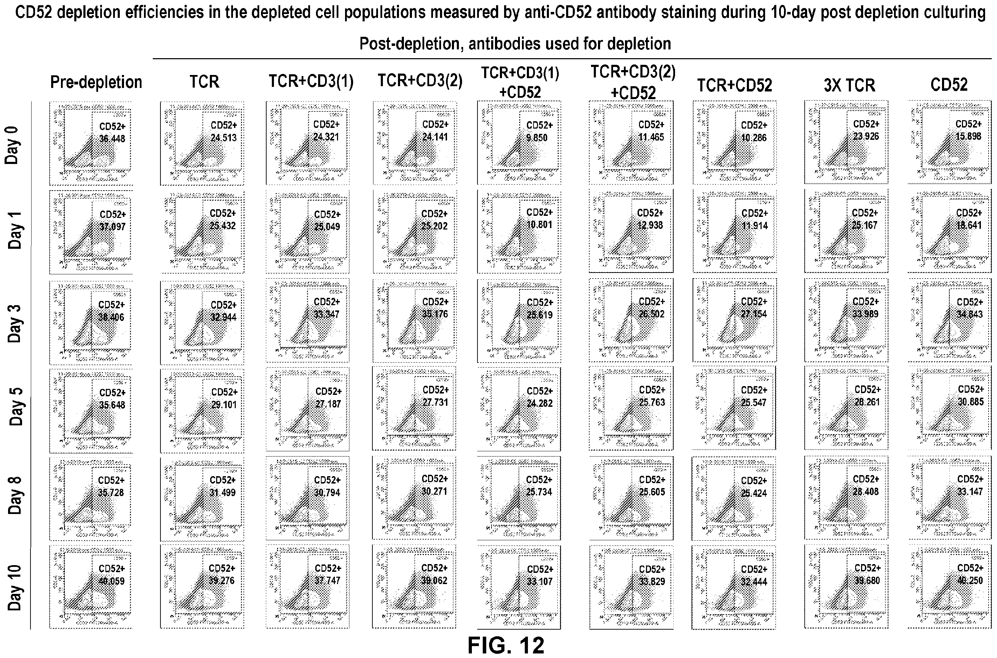

The efficiency of CD52 depletion using different combinations of antibodies disclosed herein was also studied. Specifically, anti-CD52 antibody alone or in combination with other antibodies were used for CD52 depletion, and the impact of anti-TCR and/or anti-CD3 antibodies used for TCR+ cell depletion on CD52+ cell revomal was also studied. The FACS plots of Figure 11A shows that the residual CD52+ cell frequency post depletion on Day 0 and Day 1 post depletion. The bar graph of Figure 11B shows the residual CD52+ cell frequency in a numerical manner. Finally, the FACS plots of Figure 12 shows residual CD52+ cell frequency during 10-day post depletion culturing.

Summarily, the data from Example 2 demonstrated that using both anti-TCR and anti-CD3 antibodies enhanced TCR+ cell depletion efficiency. Using an anti-CD3 antibody in addition to an anti-TCR antibody removed CD3+/TCR+ cells more extensively than, using anti-TCR antibody alone. This depletion mechanism provides an improved TCR+ cell depletion efficiency and can be a significant advantage for therapeutic allogeneic applications. The post-depleted cells showed similar growth for all the conditions during post depletion culturing, indicating that the depletion methods did not affect cell viability and growth.

## Claims

1. A method of producing a population of immune cells depleted of immune cells expressing an endogenous TCR, wherein the immune cells are genetically modified to be deficient for the endogenous TCRα or TCRβ gene in order to reduce or eliminate expression of the endogenous TCR, the method comprising:
(a) labeling a population of immune cells with an anti-TCR antibody and an anti-CD3 antibody; and
(b) separating anti-TCR antibody labeled immune cells and anti-CD3 antibody labeled immune cells from the population of immune cells.

2. A method of depleting cells expressing an endogenous TCR from a population of immune cells, wherein the immune cells are genetically modified to be deficient for the endogenous TCRα or TCRβ gene in order to reduce or eliminate expression of the endogenous TCR, the method comprising:
(a) labeling the population of immune cells with an anti-TCR antibody and an anti-CD3 antibody;
(b) separating anti-TCR antibody labeled immune cells and anti-CD3 antibody labeled immune cells from unlabeled immune cells; and
(c) collecting the unlabeled immune cells,
thereby obtaining a population of immune cells that are depleted of cells expressing the endogenous TCR.

3. The method of claim 1 or 2, further comprising:
labeling the population of immune cells with an anti-CD52 antibody; and depleting or separating anti-CD52 antibody labeled immune cells from the population of immune cells.

4. The method of any one of claims 1-3, wherein the anti-TCR antibody, anti-CD3 antibody, and/or the anti-CD52 antibody is biotin conjugated.

5. The method of claim 4, wherein the method further comprises contacting the labeled cells with an agent that specifically recognizes biotin.

6. The method of claim 5, wherein the agent that specifically recognizes biotin is selected from the group consisting of an anti-biotin antibody, avidin and streptavidin.

7. The method of claim 5 or claim 6, wherein the agent is conjugated to a magnetic bead, an agarose bead, an acoustic wave particle, a plastic welled plate, a glass welled plate, a ceramic welled plate, a column, a cell culture bag, or a membrane.

8. The method of any one of claims 1-3, wherein the anti-TCR antibody, anti-CD3 antibody, and/or the anti-CD52 antibody is directly conjugated to a support; optionally wherein the support is a magnetic bead, an agarose bead, an acoustic wave particle, a plastic welled plate, a glass welled plate, a ceramic welled plate, a column, a cell culture bag, or a membrane.

9. The method of any of claims 1-8, wherein, the separating is achieved using one of a magnetic separation, or acoustic wave separation.

10. The method of any one of the preceding claims wherein the immune cells are allogeneic immune cells.

11. The method of any one of the preceding claims, wherein the immune cells are genetically modified to reduce or eliminate expression of endogenous CD52.

12. The method of any one of the preceding claims, wherein the immune cells are engineered immune cells expressing a chimeric antigen receptor.

13. The method of any one of the preceding claims, wherein the immune cells are T cells.

14. The method of claim any one of claims 1 to 13, wherein the reduction or elimination of endogenous TCR or both endogenous TCR and endogenous CD52 expression is achieved using a TALEN, CRISPR/Cas9, a zinc finger nuclease (ZFN), a MegaTAL, a meganuclease, Cpf1, homologous recombination, or a single stranded oligodeoxynucleotide (ssODN).

15. The method of any one of claims 1- 14, wherein the population of cells that is depleted of cells expressing an endogenous TCR comprises:
(i) no more than 1.0% TCR+ cells, no more than 0.9% TCR+ cells, no more than 0.8% TCR+ cells, no more than 0.7% TCR+ cells, no more than 0.6% TCR+ cells, no more than 0.5% TCR+ cells, no more than 0.4% TCR+ cells, no more than 0.3% TCR+ cells, no more than 0.2% TCR+ cells, or no more than 0.1% TCR+ cells;or
(ii) between 0.01-0.001% TCR+ cells immediately after depletion; or
(iii) between 0.1-0.01% TCR+ cells after 1-10 days of culturing post depletion; optionally wherein the population of cells that is depleted of cells expressing an endogenous TCR comprises less than 0.1-1.0% TCR+ cells after 1 or 10 days of culturing post depletion.

16. The method of any one of claims 1-15, wherein the population of cells that is depleted of cells expressing the endogenous TCR is cryopreserved or formulated into a composition for infusion or intravenous administration, and wherein the TCR-depleted cell population expresses a chimeric antigen receptor (CAR).

## Patentansprüche

1. Verfahren zum Herstellen einer Population von Immunzellen, die an einen endogenen TCR exprimierenden Immunzellen abgereichert ist, wobei die Immunzellen gentechnisch verändert sind, sodass es ihnen an dem endogenen TCRα- oder TCRß-Gen fehlt, um so die Expression des endogenen TCR zu reduzieren oder zu eliminieren, wobei das Verfahren Folgendes umfasst:
(a) Markieren einer Population von Immunzellen mit einem Anti-TCR-Antikörper und einem Anti-CD3-Antikörper; und
(b) Trennen von mit Anti-TCR-Antikörper gekennzeichneten Immunzellen und mit Anti-CD3-Antikörper gekennzeichneten Immunzellen von der Population von Immunzellen.

2. Verfahren zum Abreichern von einen endogenen TCR exprimierenden Zellen aus einer Population von Immunzellen, wobei die Immunzellen gentechnisch modifiziert sind, sodass es ihnen an dem endogenen TCRα- oder TCRß-Gen fehlt, um so die Expression des endogenen TCR zu reduzieren oder zu eliminieren, wobei das Verfahren Folgendes umfasst:
(a) Markieren der Population von Immunzellen mit einem Anti-TCR-Antikörper und einem Anti-CD3-Antikörper;
(b) Trennen von mit Anti-TCR-Antikörper markierten Immunzellen und mit Anti-CD3-Antikörper markierten Immunzellen von nicht markierten Immunzellen; und
(c) Sammeln der nicht markierten Immunzellen,
wobei eine Population von Immunzellen erhalten wird, die an den endogenen TCR exprimierenden Zellen abgereichert ist.

3. Verfahren nach Anspruch 1 oder 2, ferner umfassend: Markieren der Population von Immunzellen mit einem Anti-CD52-Antikörper; und Abreichern oder Trennen von mit Anti-CD52-Antikörper markierten Immunzellen aus oder von der Population von Immunzellen.

4. Verfahren nach einem der Ansprüche 1-3, wobei der Anti-TCR-Antikörper, Anti-CD3-Antikörper und/oder der Anti-CD52-Antikörper biotinkonjugiert sind/ist.

5. Verfahren nach Anspruch 4, wobei das Verfahren ferner Inkontaktbringen der markierten Zellen mit einem Agens umfasst, das Biotin spezifisch erkennt.

6. Verfahren nach Anspruch 5, wobei das Agens, das Biotin spezifisch erkennt, aus der Gruppe bestehend aus einem Anti-Biotin-Antikörper, Avidin und Streptavidin ausgewählt ist.

7. Verfahren nach Anspruch 5 oder Anspruch 6, wobei das Agens an ein magnetisches Kügelchen, ein Agarosekügelchen, ein Schallwellenpartikel, eine Kunststoffplatte mit Wells, eine Glasplatte mit Wells, eine Keramikplatte mit Wells, eine Säule, einen Zellkulturbeutel oder eine Membran konjugiert ist.

8. Verfahren nach einem der Ansprüche 1-3, wobei der Anti-TCR-Antikörper, Anti-CD3-Antikörper und/oder der Anti-CD52-Antikörper direkt an ein Gerüst konjugiert ist; gegebenenfalls wobei es sich bei dem Gerüst um ein magnetisches Kügelchen, ein Agarosekügelchen, ein Schallwellenpartikel, eine Kunststoffplatte mit Wells, eine Glasplatte mit Wells, eine Keramikplatte mit Wells, eine Säule, einen Zellkulturbeutel oder eine Membran handelt.

9. Verfahren nach einem der Ansprüche 1-8, wobei das Trennen unter Verwendung entweder einer magnetischen Trennung oder einer Schallwellentrennung erreicht wird.

10. Verfahren nach einem der vorhergehenden Ansprüche, wobei es sich bei den Immunzellen um allogene Immunzellen handelt.

11. Verfahren nach einem der vorhergehenden Ansprüche, wobei die Immunzellen gentechnisch verändert sind, sodass die Expression von endogenem CD52 reduziert oder eliminiert wird.

12. Verfahren nach einem der vorhergehenden Ansprüche, wobei es sich bei den Immunzellen um konstruierte Immunzellen handelt, die einen chimären Antigenrezeptor exprimieren.

13. Verfahren nach einem der vorhergehenden Ansprüche, wobei es sich bei den Immunzellen um T-Zellen handelt.

14. Verfahren nach einem der Ansprüche 1 bis 13, wobei die Reduzierung oder Eliminierung der Expression von endogenem TCR oder sowohl endogenem TCR als auch endogenem CD52 unter Verwendung einer TALEN, CRISPR/Cas9, einer Zinkfingernuklease (ZFN), einer MegaTAL, einer Meganuklease, Cpfl, homologer Rekombination oder eines Einzelstrang-Oligodesoxynukleotids (ssODN= single stranded oligodeoxynucleotide) erreicht wird.

15. Verfahren nach einem der Ansprüche 1-14, wobei die Population von Zellen, die an einen endogenen TCR exprimierenden Zellen abgereichert ist, Folgendes umfasst:
(i) nicht mehr als 1,0 % TCR+-Zellen, nicht mehr als 0,9 % TCR+-Zellen, nicht mehr als 0,8 % TCR+-Zellen, nicht mehr als 0,7 % TCR+-Zellen, nicht mehr als 0,6 % TCR+-Zellen, nicht mehr als 0,5 % TCR+-Zellen, nicht mehr als 0,4 % TCR+-Zellen, nicht mehr als 0,3 % TCR+-Zellen, nicht mehr als 0,2 % TCR+-Zellen oder nicht mehr als 0,1 % TCR+-Zellen; oder
(ii) zwischen 0,01-0,001 % TCR+-Zellen unmittelbar nach dem Abreichern; oder
(iii) zwischen 0,1-0,01 % TCR+-Zellen nach 1-10 Tagen Kultivieren nach dem Abreichern; gegebenenfalls wobei die Population von Zellen, die an einen endogenen TCR exprimierenden Zellen abgereichert ist, weniger als 0,1-1,0 % TCR+-Zellen nach 1 oder 10 Tagen Kultivieren nach dem Abreichern umfasst.

16. Verfahren nach einem der Ansprüche 1-15, wobei die Population von Zellen, die an einen endogenen TCR exprimierenden Zellen abgereichert ist, kryokonserviert ist oder in eine Zusammensetzung zur Infusion oder intravenösen Verabreichung formuliert ist und wobei die TCR-abgereicherte Zellpopulation einen chimären Antigenrezeptor (CAR) exprimiert.

## Revendications

1. Procédé de production d'une population de cellules immunitaires appauvries en cellules immunitaires exprimant un TCR endogène, dans lequel les cellules immunitaires sont génétiquement modifiées pour être déficientes pour le gène TCRα ou TCRß endogène afin de réduire ou d'éliminer l'expression du TCR endogène, le procédé comprenant :
(a) le marquage d'une population de cellules immunitaires avec un anticorps anti-TCR et un anticorps anti-CD3 ; et
(b) la séparation de cellules immunitaires marquées par l'anticorps anti-TCR et de cellules immunitaires marquées par l'anticorps anti-CD3 à partir de la population de cellules immunitaires.

2. Procédé d'appauvrissement de cellules exprimant un TCR endogène à partir d'une population de cellules immunitaires, dans lequel les cellules immunitaires sont génétiquement modifiées pour être déficientes pour le gène TCRα ou TCRß endogène afin de réduire ou d'éliminer l'expression du TCR endogène, le procédé comprenant :
(a) le marquage de la population de cellules immunitaires avec un anticorps anti-TCR et un anticorps anti-CD3 ;
(b) la séparation de cellules immunitaires marquées par l'anticorps anti-TCR et de cellules immunitaires marquées par l'anticorps anti-CD3 de cellules immunitaires non marquées ; et
(c) la collecte des cellules immunitaires non marquées, obtenant ainsi une population de cellules immunitaires appauvries en cellules exprimant le TCR endogène.

3. Procédé selon la revendication 1 ou 2, comprenant en outre :
le marquage de la population de cellules immunitaires avec un anticorps anti-CD52 ; et l'appauvrissement ou la séparation des cellules immunitaires marquées par l'anticorps anti-CD52 de la population de cellules immunitaires.

4. Procédé selon l'une quelconque des revendications 1 à 3, dans lequel l'anticorps anti-TCR, l'anticorps anti-CD3 et/ou l'anticorps anti-CD52 est conjugué à la biotine.

5. Procédé selon la revendication 4, dans lequel le procédé comprend en outre la mise en contact des cellules marquées avec un agent qui reconnaît spécifiquement la biotine.

6. Procédé selon la revendication 5, dans lequel l'agent qui reconnaît spécifiquement la biotine est choisi dans le groupe constitué d'un anticorps anti-biotine, d'avidine et de streptavidine.

7. Procédé selon la revendication 5 ou la revendication 6, dans lequel l'agent est conjugué à une bille magnétique, une bille d'agarose, une particule d'onde acoustique, une plaque à puits en plastique, une plaque à puits en verre, une plaque à puits en céramique, une colonne, un sac de culture cellulaire ou une membrane.

8. Procédé selon l'une quelconque des revendications 1 à 3, dans lequel l'anticorps anti-TCR, l'anticorps anti-CD3 et/ou l'anticorps anti-CD52 est directement conjugué à un support ; éventuellement dans lequel le support est une bille magnétique, une bille d'agarose, une particule d'onde acoustique, une plaque à puits en plastique, une plaque à puits en verre, une plaque à puits en céramique, une colonne, un sac de culture cellulaire ou une membrane.

9. Procédé selon l'une quelconque des revendications 1 à 8, dans lequel la séparation est réalisée en utilisant l'une parmi une séparation magnétique ou une séparation par onde acoustique.

10. Procédé selon l'une quelconque des revendications précédentes, dans lequel les cellules immunitaires sont des cellules immunitaires allogéniques.

11. Procédé selon l'une quelconque des revendications précédentes, dans lequel les cellules immunitaires sont génétiquement modifiées pour réduire ou éliminer l'expression de CD52 endogène.

12. Procédé selon l'une quelconque des revendications précédentes, dans lequel les cellules immunitaires sont des cellules immunitaires modifiées exprimant un récepteur antigénique chimérique.

13. Procédé selon l'une quelconque des revendications précédentes, dans lequel les cellules immunitaires sont des cellules T.

14. Procédé selon l'une quelconque des revendications 1 à 13, dans lequel la réduction ou l'élimination du TCR endogène ou à la fois de l'expression du TCR endogène et du CD52 endogène est réalisée en utilisant un TALEN, CRISPR/Cas9, une nucléase à doigt de zinc (ZFN), un MegaTAL, une méganucléase, Cpfl, une recombinaison homologue ou un oligodésoxynucléotide simple brin (ssODN).

15. Procédé selon l'une quelconque des revendications 1 à 14, dans lequel la population de cellules qui est appauvrie en cellules exprimant un TCR endogène comprend :
(i) pas plus de 1,0 % de cellules TCR+, pas plus de 0,9 % de cellules TCR+, pas plus de 0,8 % de cellules TCR+, pas plus de 0,7 % de cellules TCR+, pas plus de 0,6 % de cellules TCR+, pas plus de 0,5 % de cellules TCR+, pas plus de 0,4 % de cellules TCR+, pas plus de 0,3 % de cellules TCR+, pas plus de 0,2 % de cellules TCR+ ou pas plus de 0,1 % de cellules TCR+ ; ou
(ii) entre 0,01 et 0,001 % de cellules TCR+ immédiatement après l'appauvrissement ; ou
(iii) entre 0,1 et 0,01 % de cellules TCR+ après 1 à 10 jours de culture post-appauvrissement ; éventuellement, dans lequel la population de cellules qui est appauvrie en cellules exprimant un TCR endogène comprend moins de 0,1 à 1,0 % de cellules TCR+ après 1 ou 10 jours de culture post-appauvrissement.

16. Procédé selon l'une quelconque des revendications 1 à 15, dans lequel la population de cellules qui est appauvrie en cellules exprimant le TCR endogène est cryoconservée ou formulée dans une composition pour perfusion ou administration intraveineuse, et dans lequel la population de cellules appauvrie en TCR exprime un récepteur antigénique chimérique (CAR).
